(19) Europäisches Patentamt
European Patent Office
Office européen des brevets

(11) **EP 3 964 590 B1**

(12) **EUROPEAN PATENT SPECIFICATION**

(45) Date of publication and mention
of the grant of the patent:
**18.02.2026 Bulletin 2026/08**

(21) Application number: **20802982.7**

(22) Date of filing: **28.04.2020**

(51) International Patent Classification (IPC):
***C12Q 1/6886*** (2018.01)    ***G16B 20/00*** (2019.01)
***G16B 25/10*** (2019.01)

(52) Cooperative Patent Classification (CPC):
**C12Q 1/6886; G16B 25/10;** C12Q 2600/118;
C12Q 2600/158; G16H 50/30

(86) International application number:
**PCT/KR2020/005624**

(87) International publication number:
**WO 2020/226333 (12.11.2020 Gazette 2020/46)**

(54) **METHOD OF PREDICTING CANCER PROGNOSIS AND COMPOSITION FOR SAME**

VERFAHREN ZUR VORHERSAGE EINER KREBSPROGNOSE UND ZUSAMMENSETZUNG DAFÜR

MÉTHODE DE PRÉDICTION ET DE PRONOSTIC DU CANCER ET COMPOSITION À CET EFFET

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.05.2019 KR 20190052364**

(43) Date of publication of application:
**09.03.2022 Bulletin 2022/10**

(73) Proprietor: DCGEN Co., Ltd.
**Seoul 03170 (KR)**

(72) Inventors:
• **HAN, Won Shik
Seoul 05572 (KR)**
• **LEE, Han Byoel
Seoul 06084 (KR)**
• **PARK, In Ae
Seoul 06334 (KR)**
• **RYU, Han Suk
Seoul 06045 (KR)**
• **AHN, Sei Hyun
Seoul 05505 (KR)**
• **LEE, Jong Won
Seoul 05505 (KR)**
• **LEE, Sae Byul
Seoul 05505 (KR)**

• **LEE, Hee Jin
Seoul 05505 (KR)**
• **KIM, Ae Ree
Seoul 06010 (KR)**
• **KIM, Chung Yeul
Uijeongbu-si Gyeonggi-do 11636 (KR)**
• **YOON, Sung Roh
Seoul 08826 (KR)**
• **KIM, Sun
Seoul 08826 (KR)**
• **KWON, Sun Young
Seoul 08826 (KR)**
• **KIM, Min Su
Seoul 08826 (KR)**
• **JO, Jeong Hee
Seoul 08826 (KR)**

(74) Representative: **Schnappauf, Georg
ZSP Patentanwälte PartG mbB
Hansastraße 32
80686 München (DE)**

(56) References cited:
KR-A- 20130 023 312    KR-A- 20180 052 985
KR-A- 20180 052 985    KR-A- 20190 024 923
US-A1- 2015 218 648

- GUNHEE LEE ET AL: "Classification of Genes Based on Age-Related Differential Expression in Breast Cancer", GENOMICS & INFORMATICS, vol. 15, no. 4, 29 December 2017 (2017-12-29), Korea, pages 156 - 161, XP055613932, ISSN: 2234-0742, DOI: 10.5808/GI.2017.15.4.156
- COLLIGNON OLIVIER ET AL: "Comparison of the modified unbounded penalty and the LASSO to select predictive genes of response to chemotherapy in breast cancer", PLOS ONE, vol. 13, no. 10, 1 October 2018 (2018-10-01), pages e0204897, XP093046433, DOI: 10.1371/journal.pone.0204897
- LIU MIN ET AL: "Identification of genes associated with survival of breast cancer patients", BREAST CANCER, JAPANESE BREAST CANCER SOCIETY, TOKYO, JP, vol. 26, no. 3, 19 October 2018 (2018-10-19), pages 317 - 325, XP036767396, ISSN: 1340-6868, [retrieved on 20181019], DOI: 10.1007/S12282-018-0926-9
- "AFFYMETRIX CATALOG; PRODUKT: HUMAN GENOME U133A ARRAY; ARRAY FINDER", AFFYMETRIX PRODUCT CATALOG, XX, XX, 1 July 2002 (2002-07-01), pages 1, XP002267612
- MOLLY E KLEIN , DAVID J DABBS, YONGLI SHUAI, ADAM M BRUFSKY, RACHEL JANKOWITZ, SHANNON L PUHALLA, ROHIT BHARGAVA: "Prediction of the Oncotype DX recurrence score: use of pathology-generated equations derived by linear regression analysis", MODEM PATHOLOGY, vol. 26, no. 5, 2013, pages 658 - 664, XP055759430, DOI: 10.1038/modpathol.2013.36

## Description

### Technical Field

The present disclosure relates to a method for predicting breast cancer prognosis and a device for use in determining breast cancer prognosis. More specifically, the present disclosure relates to a method for predicting whether chemotherapy has a therapeutic effect and a device therefor.

### Background Art

Cancer is one of the most common causes of death worldwide. About 10 million new cases of cancer occur each year, accounting for about 12% of all causes of deaths, which is the third leading cause of death. Among various types of cancer, breast cancer, especially estrogen hormone receptor-positive (ER+) breast cancer without axillary lymph node metastasis, which accounts for about half of breast cancer patients, has a 10-year recurrence rate of 15% even with only 5 years of anti-hormonal treatment, and when anticancer chemotherapy is added, the absolute value of the 10-year recurrence rate is reduced to about 5% (Fisher et al, Lancet. 10;364(9437):858-68, PMID: 15351193). However, with the development of the Oncotype Dx test in 2004, it has been found that only some patients need chemotherapy (see Paik et al, J Clin Oncol 24(23):3726-34, PMID: 16720680).

Since Oncotype Dx was developed, many tests for predicting breast cancer prognosis based on transcriptional gene analysis, such as Mammaprint, Endopredict, Breast Cancer Index, and Prosigna, have been developed and commercialized, but Oncotype Dx is the only test that has demonstrated clinical applicability for the decision of whether or not to use chemotherapy (US 2015-0079591 A1 and Paik et al, J Clin Oncol 24(23):3726-34, PMID: 16720680).

On the other hand, this Oncotype Dx is expensive in terms of cost, and it was confirmed that the Oncotype Dx does not allow analysis in all age groups (see Wkilliams AD et al, Ann Surg Oncol. 2018 Oct;25(10):2875-2883. doi: 10.1245/s10434-018-6600-9).

KR 2018 0052985 A discloses a method for predicting the prognosis of breast cancer. Gunhee Lee & Minho Lee, Genomics & Informatics, 15 (2017): 156-161 disclose and classify breast cancer-related genes. Collignon Olivier et al, Plos One, 13 (2018) is concerned with the selection of predictive genes to chemotherapy in breast cancer patients, and Liu Min et al, Breast Cancer, 26 (2019): 317-325 is concerned with the identification of genes associated with survival of breast cancer patients.

Therefore, the present inventors have discovered a device and a method for predicting breast cancer prognosis and deciding whether to use chemotherapy, which allow analysis in all age groups, thereby leading to the present disclosure.

## DISCLOSURE

The present invention is defined by the appended claims.

### Technical Problem

An object of the present disclosure is to provide a device capable of predicting breast cancer prognosis and deciding whether to use chemotherapy, which is inexpensive and enables analysis for all age groups.

### Technical Solution

Hereinafter, various embodiments described herein will be described with reference to the accompanying drawings. In the following description, numerous specific details are set forth, such as specific configurations, compositions, and processes, etc., in order to provide a thorough understanding of the present disclosure. However, certain embodiments may be practiced without one or more of these specific details, or in combination with other known methods and configurations. In other instances, well-known processes and manufacturing techniques have not been described in particular detail in order to not unnecessarily obscure the present disclosure. Reference throughout this specification to "one embodiment" or "an embodiment" means that a particular feature, configuration, composition, or characteristic described in connection with the embodiment is included in at least one embodiment of the present disclosure. Thus, the appearances of the phrase "in one embodiment" or "an embodiment" in various places throughout this specification are not necessarily referring to the same embodiment of the present disclosure. Additionally, the particular features, configurations, compositions, or characteristics may be combined in any suitable manner in one or more embodiments.

Unless otherwise stated in the specification, all the scientific and technical terms used in the specification have the same meanings as commonly understood by those skilled in the technical field to which the present disclosure pertains.

In the present specification, the term "polynucleotide", when used in singular or plural, generally refers to any polyribonucleotide or polydeoxyribonucleotide, which may be unmodified RNA or DNA or modified RNA or DNA. Thus, for instance, polynucleotides as defined in the present specification include, but are not limited to, single- and double-stranded DNA, DNA including single- and double-stranded regions, single- and double-stranded RNA, and RNA including single- and double-stranded regions, and hybrid molecules comprising DNA and RNA that may be single-stranded or, more typically, double-stranded or may include single- and double-stranded regions. In addition, the term "polynucleotide" as used in the present specification refers to triplestranded regions comprising RNA or DNA or both RNA and DNA. The strands in such regions may be derived from the same molecule or from different molecules. These regions may include all of one or more of the molecules, but more typically involve only a region of some of the molecules. One of the molecules of a double-helical region is an oligonucleotide. The term "polynucleotide" specifically includes cDNAs. The term includes DNAs (including cDNAs) and RNAs that contain one or more modified bases. Thus, DNAs or RNAs with backbones modified for stability or for other reasons are "polynucleotides" as the term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically or metabolically modified forms of unmodified polynucleotides, as well as the chemical forms of DNA and RNA characteristic of viruses and cells.

The term "oligonucleotide" refers to a relatively short polynucleotide, including, without limitation, single-stranded deoxyribonucleotides, single- or double-stranded ribonucleotides, RNA:DNA hybrids and double-stranded DNAs. Oligonucleotides, such as single-stranded DNA probe oligonucleotides, are synthesized by chemical methods, for example using automated oligonucleotide synthesizers that are commercially available. In addition, oligonucleotides can be made by a variety of other methods, including *in vitro* recombinant DNA-mediated techniques and by expression of DNAs in cells and organisms.

The term "gene expression" refers to the conversion of the DNA gene sequence information into transcribed RNA (the initial unspliced RNA transcript or the mature mRNA) or the encoded protein product. Gene expression can be monitored by measuring the levels of either the entire RNA or protein products of the gene or their subsequences.

The term "over-expression" with regard to an RNA transcript is used to refer to the level of the transcript determined by normalization to the level of reference mRNAs, which might be all measured transcripts in a sample or a particular reference set of mRNAs.

The term "gene amplification" refers to a process by which multiple copies of a gene or gene fragment are formed in a particular cell or cell line. The duplicated region (amplified DNA) is also referred to as "amplicon." Usually, the amount of mRNA produced, that is, the level of gene expression, also increases in the proportion of the number of copies made of each gene expressed.

The term "prognosis" as used in the present specification refers to the progress and cure of diseases, such as cancer migration and invasion in tissues, metastasis to other tissues, and death caused by disease. For the purposes of the present disclosure, prognosis refers to the prognosis of disease progression or survival of a breast cancer patient. Using the method of the present disclosure, it is possible to easily determine the survival prognosis for breast cancer patients, and thus it is possible to easily decide whether to use an additional therapeutic method. Ultimately, it is possible to improve the survival rate after the onset of breast cancer.

The term "prediction" as used in the present specification refers to an action that predicts the course and outcome of a disease by determining the response of a patient to a drug or a set of drugs. More specifically, the term "prognostic prediction" may be interpreted as referring to any action that predicts the course of a disease after treatment by considering the patient's conditions comprehensively, because the course of disease after treatment may vary depending on the physiological or environmental conditions of the patient.

The term "beneficial response" as used in the present disclosure means an improvement in any measure of patient status including those measures ordinarily used in the art such as overall survival, long-term survival, recurrence-free survival, and distant recurrence-free survival. Recurrence-free survival (RFS) refers to the time (in months) from surgery to the first local, regional, or distant recurrence. Distant recurrence-free survival (DRFS) or distant metastasis-free survival (DMFS) refers to the time (in months) from surgery to the first distant recurrence. Recurrence refers to RFS and/or DFRS. The term "long-term" survival as used in the present specification refers to survival for at least 3 years, or at least 5 years, or at least 8 years, or at least 10 years following surgery or other treatment.

The term "tumor," as used in the present specification, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. According to the present invention, the cancer is breast cancer.

The "pathology" of cancer includes all phenomena that compromise the health of the patient. Examples thereof include, but are not limited to, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc.

The term "decision index (DI)" as used in the present specification refers to a score that can determine whether the prognosis of cancer in a subject is good or poor, including the concepts of prognostic factors and treatment predictors. In breast cancer, prognostic factors differ from treatment predictive factors. Prognostic factors are variable related to the natural history of breast cancer and affect the recurrence rate and outcome of patients with breast cancer. Clinical parameters associated with poor prognosis include, for example, lymph node involvement, tumor enlargement, and high-grade (malignant) tumors. Prognostic factors may be effectively used to classify patients into subgroups with different basic risks of recurrence. In contrast, treatment predictive factors are variables related to the likelihood that each patient will respond favorably to treatment, such as antiestrogen therapy or chemotherapy, and are unrelated to prognosis.

In the present specification, nucleic acid (including DNA or RNA) sequencing may be next-generation sequencing (NGS). The term "nucleic acid sequencing" may be used interchangeably with the term "base sequencing" or "sequencing". The term "NGS" may be used interchangeably with the term "massive parallel sequencing" or "secondgeneration sequencing". The NGS is a technique for simultaneous sequencing of a large amount of nucleic acid fragments, which may include fragmenting the whole genome in a chip-based and polymerase chain reaction (PCR)-based paired end format and performing sequencing at an ultra-high speed based on hybridization of the fragments. The NGS may be performed by, for example, 454 platform (Roche), GS FLX titanium, Illumina MiSeq, Illumina HiSeq, Illumina HiSeq 2500, Illumina Genome Analyzer, Solexa platform, SOLiD System (Applied Biosystems), Ion Proton (Life Technologies), Complete Genomics, Helicos Biosciences Heliscope, Pacific Biosciences single-molecule real-time (SMRT™) technology, or combinations thereof. The nucleic acid sequencing may be a nucleic acid sequencing method for analyzing only a region of interest. The nucleic acid sequencing may include, for example, NGS-based targeted sequencing, targeted deep sequencing, or panel sequencing.

As used herein, "next-generation sequencing (NGS)" refers to a method of decomposing a genome into countless fragments, reading genetic information of each fragment, combining the genetic information, and then analyzing the entire nucleotide sequence. NGS is advantageously capable of high-throughput genome sequencing, and is also referred to as high-throughput sequencing, massive parallel sequencing, or secondgeneration sequencing. Compared to the NGS, the entire human genome can also be read by Sanger sequencing, but in this case, only known genes can be targeted, and hence the test is limited, and repeated experiments are required to test multiple genes. For example, next-generation sequencing is an analysis method that has been greatly improved in terms of time and cost, compared to Sanger sequencing which needs to be carried out about 3 million times. Massive parallel sequencing made possible by next-generation sequencing (NGS) technology is another way to approach the enumeration of RNA transcripts in a tissue sample and RNA-seq is a method that utilizes this. It is currently the most powerful analytical tool used for transcriptome analyses, including gene expression level difference between different physiological conditions, or changes that occur during development or over the course of disease progression. Specifically, RNA-seq can be used to study phenomena such as gene expression changes, alternative splicing events, allele-specific gene expression, and chimeric transcripts, including gene fusion events, novel transcripts and RNA editing.

As used in the present specification, the term "probe" in a broad sense includes a probe that is attached specifically to a target gene to identify and/or detect the target gene.

As used in the present specification, "breast cancer" means, for example, those conditions classified by biopsy as malignant pathology. The clinical delineation of breast cancer diagnoses is well-known in the medical arts. Those skilled in the art will appreciate that breast cancer refers to any malignancy of the breast tissue, including, for example, carcinomas and sarcomas. In particular embodiments, breast cancer is ductal carcinoma in situ (DCIS), lobular carcinoma in situ (LCIS), or mucinous carcinoma. Breast cancer also refers to infiltrating ductal carcinoma (IDC) or infiltrating lobular carcinoma (ILC). In most embodiments of the present disclosure, the subject of interest is a human patient suspected of or actually diagnosed with breast cancer.

"Early-stage breast cancer" means stages 0 (*in situ* breast cancer), I (T1, N0, M0), IIA (T0-1, N1, M0 or T2, N0, M0), and IIB (T2, N1, M0 or T3, N0, M0). Early-stage breast cancer patients exhibit little or no lymph node involvement. As used herein, "lymph node involvement" or "lymph node status" refers to whether the cancer has metastasized to the lymph nodes. Breast cancer patients are classified as "lymph nodepositive" or "lymph node-negative" on this basis. Methods of identifying breast cancer patients and staging the disease are well known and may include manual examination, biopsy, review of patient's and/or family history, and imaging techniques, such as mammography, magnetic resonance imaging (MRI), ultrasonography, computed tomography (CT), and positron emission tomography (PET).

In addition, breast cancer is managed by several alternative strategies that may include, for example, surgery, radiation therapy, hormone therapy, chemotherapy, or some combination thereof. As is known in the art, treatment decisions for individual breast cancer patients can be based on the number of lymph nodes involved, estrogen and progesterone receptor status, size of the primary tumor, and stage of the disease at diagnosis. Analysis of a variety of clinical factors and clinical trials has led to the development of recommendations and treatment guidelines for early-stage breast cancer by the International Consensus Panel of the St. Gallen Conference (2001). See Goldhirsch et al. (200I) J.

Clin. Oncol. 19:3817-3827.

As used in the present specification, the term "subject" refers to a patient who has developed or is suspected of having developed breast cancer, and may mean a patient in need of or expected to require appropriate treatment for breast cancer, but is not limited thereto.

As used in the present specification, the term "biological sample" means any sample enabling the identification of the patient's genetic information, and may include, but is not limited to, blood, plasma, serum, etc., and may be, but is not limited to, any type of sample enabling gene identification.

As used in the present specification, the term "diagnostic device" refers to a device capable of diagnosing diseases *in vitro* based on substances generated in the human body, such as blood, saliva, and urine. For example, the diagnostic device may be, but is not limited to, any type of device that includes a detection unit, an arithmetic unit, and an output unit, and is capable of analyzing a gene from the above substances.

According to the present invention, there is provided a method for determining breast cancer prognosis, the method including steps of: (a) measuring the expression levels of a first gene group, a second gene group, a third gene group, a fourth gene group, a fifth gene group and a sixth gene group in a biological sample obtained from a subject, and normalizing the measured expression levels; and (b) calculating a decision index (DI) by multiplying the expression level of each of the first gene group, the second gene group, the third gene group, the fourth gene group, the fifth gene group and the sixth gene group, normalized in step (a), by a regression coefficient value, and summing the multiplied values, thereby predicting breast cancer prognosis in the subject.

A step of measuring the expression level of each of the first gene group, the second gene group, the third gene group, the fourth gene group, the fifth gene group, and the sixth gene group for the biological sample obtained from the subject may be performed first.

The first gene group consists of ESR1 (estrogen receptor 1), PGR (progesterone receptor), and SCUBE2 (signal peptide, CUB domain and EGF like domain containing 2).

The second gene group consists of CTSL2 (cathepsin V), and MMP11 (matrix metallopeptidase 11).

The third gene group consists of TFRC (transferrin receptor), and CX3CR1 (C-X3-C motif chemokine receptor 1).

The fourth gene group consists of KIF14 (kinesin family member 14), RRM2 (ribonucleotide reductase regulatory subunit M2), SHCBP1 (SHC binding and spindle associated 1), SLC7A5 (solute carrier family 7 member 5), and KPNA2 (karyopherin subunit alpha 2).

The fifth gene group consists of AURKA (aurora kinase A), CCNE2 (cyclin E2), CENPE (centromere protein E), E2F8 (E2F transcription factor 8), KIF18A (kinesin family member 18A), and KIF23 (kinesin family member 23).

The sixth gene group consists of JMJD5 (lysine demethylase 8), CACNA1D (calcium voltage-gated channel subunit alpha1 D), and GSTM1 (glutathione S-transferase Mu 1).

As the first gene group, the second gene group, the third gene group, the fourth gene group, the fifth gene group, or the sixth gene group includes a plurality of genes, the step of measuring the expression level is performed by measuring the expression level of each of the plurality of genes belonging to each gene group.

The measurement of the expression level according to the present disclosure is a process of identifying the presence and expression level of mRNA of the gene group, and may be performed by measuring the expression level of a corresponding gene from mRNA extracted from the sample obtained from the subject. Analysis methods for measuring the expression levels include, but are not limited to, RT-PCR, competitive RT-PCR, real-time RT-PCR, RNase protection assay (RPA), northern blotting, and DNA microarray chip assay, and measurement of the expression levels may be performed by any appropriate method that is commonly used in the art.

An agent for measuring the expression level of mRNA is preferably an antisense oligonucleotide, a primer, or a probe. Either a primer that specifically amplifies a specific region of each of these genes or a probe may be designed based on the nucleotide sequence of each gene group. Since the nucleotide sequence of each gene group according to the present disclosure is registered in GenBank and is known in the art, those skilled in the art can design an antisense oligonucleotide or primer capable of specifically amplifying a specific region of each of these genes, or a probe, based on the nucleotide sequence.

After the expression levels of the first gene group, the second gene group, the third gene group, the fourth gene group, the fifth gene group and the sixth gene group are measured as described above, a step of normalizing the measured expression level of each of the first gene group, the second gene group, the third gene group, the fourth gene group, the fifth gene group and the sixth gene group is performed.

The normalization may be performed by measuring the expression level of a reference gene, and then normalizing each of the expression level of the first gene group, the expression level of the second gene group, the expression level of the third gene group, the expression level of the fourth gene group, the expression level of the fifth gene group, and the expression level of the sixth gene group to the measured expression level of the reference gene.

As a method for calculating the expression levels of differentially expressed genes according to the present disclosure, the expression level of each gene or transcript in each sample may be identified using the number of mapped reads through RNA sequencing. When the expression level is defined by the number of mapped reads, there may be an error for each sample, and it is difficult to consider the number of reads as an objective value. Hence, more preferably, a normalization process is performed as a method for obtaining an objective value.

Typically, methods that are mainly used in the art to analyze RNA sequence expression include various methods of calculating and normalizing values such as FPKM (Fragments Per Kilobase of transcripts per Million mapped reads), RPKM (Reads Per Kilobase of transcript per Million mapped reads), TPM (Transcripts Per Million), or TMM (Trimmed Mean of M-value).

Among conventional normalization techniques that have been generally used in the art, the TMM (Trimmed Mean of M-value) technique that is used in R package edgeR (Robinson et al. Bioinformatics 2010) is known to have the highest stability (Dillies et al. Briefings in bioinformatics 2013). In the present disclosure, it is possible to design and use a pipeline for automatically extracting normalized gene expression information from the target RNA sequencing data generated using the edgeR package. The sequencing data generated using NGS technology are mapped to a reference genome using common alignment software (RNA-STAR) (Dobin et al. Bioinformatics 2013), and the number of sequences from each gene is counted through the mapping result, and a direct estimate of the expression level of each gene is extracted. The data mapped in the BAM file format are input into the developed normalization pipeline, and thus the mapped data may be calculated as normalized expression level values, which may be compared between samples, by a series of software packages built into the pipeline (htseq-count (Anders et al. Bioinformatics 2014), edgeR (Robinson et al. Bioinformatics 2010)).

More specifically, the "normalization process" in the present disclosure further improves stability by removing low-quality reads and artificially generated reads among the reads generated using NGS technology, mapping the remaining reads to a human reference genome sequence using STAR aligner software, and extracting only conserved exons with the mapped BAM file format. After the above process, the expression level of each gene is quantified using htseq-count. The quantified expression level is obtained using the TMM (Trimmed Mean of M-value) technique, which is used in R package edgeR, among conventional normalization techniques, but in particular, is normalized to the standard patient group, and this modified TMM technique has higher stability than other conventional normalization techniques. Through this series of processes, the quantified expression level can be calculated as a normalized expression level value that can be compared between samples. However, the normalization process is not limited thereto as long as it corresponds to a normalization method that is commonly used in the art.

In the present disclosure, the term "reference gene" refers to all transcripts that are maintained at approximately the same level regardless of the cell or tissue type, or the presence of a confounding agent (i.e., a confounder). In the present disclosure, the reference gene may be useful as an internal control for normalizing gene expression data.

Although the type of the reference gene in the present disclosure is not particularly limited, but the reference gene may be, for example, any one or more selected from the group consisting of ACTB, APOBEC3B, ASF1B, ASPM, AURKB, BAG1, BCL2, BIRC5, BLM, BUB1, BUB1B, C14orf45, C16orf61, C7orf63, CCNA2, CCNB1, CCNB2, CCNE1, CCT5, CD68, CDC20, CDC25A, CDC45, CDC6, CDCA3, CDCA8, CDK1, CDKN3, CENPA, CENPF, CENPM, CENPN, CEP55, CHEK1, CIRBP, CKS2, CRIM1, CYBRD1, DBF4, DDX39, DLGAP5, DNMT3B, DONSON, DTL, E2F1, ECHDC2, ERBB2, ERCC6L, ESPL1, EXO1, EZH2, FAM64A, FANCI, FBXO5, FEN1, FOXM1, GAPDH, GINS1, GRB7, GTSE1, GUSB, HJURP, HMMR, HN1, IFT46, KIF11, KIF15, KIF18B, KIF20A, KIF2C, KIF4A, KIFC1, LMNB1, LMNB2, LRIG1, LRRC48, LRRC59, MAD2L1, MARCH8, MCM10, MCM2, MCM6, MELK, MKI67, MLF1IP, MYBL2, NCAPG, NCAPG2, NCAPH, NDC80, NEK2, NUP93, NUSAP1, OIP5, PBK, PDSS1, PKMYT1, PLK1, PLK4, PRC1, PTTG1, RACGAP1, RAD51, RAD51AP1, RAI2, RFC4, RPLPO, SETBP1, SF3B3, SHMT2, SLC25A12, SPAG5, SPC25, SQLE, STARD13, STIL, STMN1, SYNC, TACC3, TK1, TOP2A, TPX2, TRIP13, TROAP, TTK, UBE2C, UBE2S, ZWINT, C10orf76, C12orf72, CIAO1, CNOT4, DBR1, DND1, FBXO42, GRK4, HNRNPK, HNRNPL, HNRNPR, KHDRBS1, KLRAQ1, LACE1, LOC148189, LOC285033, LOC493754, MRPL44, NRF1, PKNOX1, PPHLN1, RRN3P3, SENP8, SLC4A1AP, TARDBP, THRAP3, TTLL11, WDR33, and ZNF143.

In the present disclosure, as the first gene group, the second gene group, the third gene group, the fourth gene group, the fifth gene group, or the sixth gene group includes a plurality of genes, the normalization is performed for each of the plurality of genes belonging to each gene group.

In the present disclosure, after the expression level of each of the first gene group, the second gene group, the third gene group, the fourth gene group, the fifth gene group, and the sixth gene group is normalized as described above, a step of calculating a decision index (DI) by multiplying the normalized expression level of each of the first gene group, the second gene group, the third gene group, the fourth gene group, the fifth gene group and the sixth gene group by a regression coefficient (weight) value and summing the multiplied values is performed.

In the present disclosure, as the first gene group, the second gene group, the third gene group, the fourth gene group, the fifth gene group, or the sixth gene group includes a plurality of genes, the normalized expression level of each of the plurality of genes belonging to each gene group are multiplied by a regression coefficient value. In this case, even if a plurality of genes belong to the same gene group, the regression coefficient values multiplied by the respective normalized expression levels may be the same as or different from each other.

In the present disclosure, the decision index (DI) is obtained by multiplying the normalized expression level of each of the first gene group, the second gene group, the third gene group, the fourth gene group, the fifth gene group and the sixth gene group by the regression coefficient value and then summing the multiplied values. A correction coefficient value is additionally added.

As described herein, the correction coefficient value may be a rational number ranging from 35.09 to 47.07. According to the present invention, the correction coefficient value is 41.16.

As described herein, the decision index (DI) may be expressed by Equation 1 as follows:

[Equation 1]

$$DI = aX(q)+bY(r)+cZ(s)+dK(t)+eL(u)+fM(v)$$

wherein

q, r, s, t, u and v are each independently an integer of 1 or more, preferably an integer ranging from 1 to 6. More preferably, q may be an integer ranging from 1 to 3, r may be an integer ranging from 1 to 2, s may be an integer ranging from 1 to 2, t may be an integer ranging from 1 to 5, u may be an integer ranging from 1 to 6, and v may be an integer ranging from 1 to 3.

a is a regression coefficient value multiplied by the normalized expression level of the first gene group, and may be a rational number ranging from -2.36 to -0.34.

b is a regression coefficient value multiplied by the normalized expression level of the second gene group, and may be a rational number ranging from 0.17 to 0.42.

c is a regression coefficient value multiplied by the normalized expression level of the third gene group, and may be a rational number ranging from -0.06 to 0.22.

d is a regression coefficient value multiplied by the normalized expression level of the fourth gene group, and may be a rational number ranging from 0.01 to 0.73.

e is a regression coefficient value multiplied by the normalized expression level of the fifth gene group, and may be a rational number ranging from 0.08 to 0.65.

f is a regression coefficient value multiplied by the normalized expression level of the sixth gene group, and may be a rational number ranging from -0.58 to -0.02.

X(q) may be the sum of the normalized expression levels of q genes belonging to the first gene group. However, when q is 1, X(q) may be a value of the normalized expression level of any one gene belonging to the first gene group. In addition, aX(q) may be the sum of the values obtained by multiplying the normalized expression level of each of q genes belonging to the first gene group by a, and when q is an integer of 2 or more, a values multiplied by the normalized expression level of each gene may be the same as or different from each other.

Y(r) may be the sum of the normalized expression levels of r genes belonging to the second gene group. However, when r is 1, Y(r) may be a value of the normalized expression level of any one gene belonging to the second gene group. In addition, bY(r) may be the sum of the values obtained by multiplying the normalized expression level of each of r genes belonging to the second gene group by b, and when r is an integer of 2 or more, b values multiplied by the normalized expression level of each gene may be the same as or different from each other.

Z(s) may be the sum of the normalized expression levels of s genes belonging to the third gene group. However, when s is 1, Z(s) may be a value of the normalized expression level of any one gene belonging to the third gene group. In addition, cZ(s) may be the sum of the values obtained by multiplying the normalized expression level of each of s genes belonging to the third gene group by c, and when s is an integer of 2 or more, c values multiplied by the normalized expression level of each gene may be the same as or different from each other.

K(t) may be the sum of the normalized expression levels of t genes belonging to the fourth gene group. However, when t is 1, K(t) may be a value of the normalized expression level of any one gene belonging to the fourth gene group. In addition, dK(t) may be the sum of the values obtained by multiplying the normalized expression level of each of t genes belonging to the fourth gene group by d, and when t is an integer of 2 or more, d values multiplied by the normalized expression level of each gene may be the same as or different from each other.

L(u) may be the sum of the normalized expression levels of u genes belonging to the fifth gene group. However, when u is 1, L(u) may be a value of the normalized expression level of any one gene belonging to the fifth gene group. In addition, the eL (u) may be the sum of values obtained by multiplying the normalized expression level of each of u genes belonging to the fifth gene group by e, and when u is an integer of 2 or more, e values multiplied by the normalized expression level of each gene may be the same as or different from each other.

M(v) may be the sum of the normalized expression levels of v genes belonging to the sixth gene group. However, when v is 1, M(v) may be a value of the normalized expression level of any one gene belonging to the sixth gene group. In addition, fM(v) may be the sum of values obtained by multiplying the normalized expression level of each of v genes belonging to the sixth gene group by f, and when v is an integer of 2 or more, f values multiplied by the normalized expression level of each gene may be the same or different from each other.

As described herein, more specifically, aX(q) may be expressed by Equation 2 below.

[Equation 2]

$$aX(q) = a_1*X_1+ \ldots + a_q*X_q$$

wherein

q is an integer of 1 or more, preferably an integer ranging from 1 to 3,
$a_1$ to $a_q$ are each independently a rational number ranging from -2.36 to -0.34,
$X_1$ to $X_q$ may each independently be the normalized expression level of any gene belonging to the first gene group.

Also, as described herein, bY(r) may be expressed by Equation 3 below.

[Equation 3]

$$bY(r) = b_1*Y_1+ \ldots + b_q*Y_q$$

wherein

r is an integer of 1 or more, preferably an integer ranging from 1 to 2,
$b_1$ to $b_q$ are each independently a rational number ranging from 0.17 to 0.42,
$Y_1$ to $Y_q$ may each independently be the normalized expression level of any gene belonging to the second gene group.

In addition, as described herein, cZ(s) may be expressed by Equation 4 below.

[Equation 4]

$$cZ(s) = c_1*Z_1+ \ldots + c_q*Z_q$$

wherein

s is an integer of 1 or more, preferably an integer ranging from 1 to 2,
$c_1$ to $c_q$ are each independently a rational number ranging from -0.06 to 0.22, and
$Z_1$ to $Z_q$ may each independently be the normalized expression level of any gene belonging to the third gene group.

In addition, as described herein, the dK(t) may be expressed by Equation 5 below.

[Equation 5]

$$dK(t) = d_1*K_1+ \ldots + d_q*K_q$$

wherein

t is an integer of 1 or more, preferably an integer ranging from 1 to 5,
$d_1$ to $d_q$ are each independently a rational number of 0.01 to 0.73, and
$K_1$ to $K_q$ may each independently be the normalized expression level of any gene belonging to the fourth gene group.

In addition, as described herein, eL(u) may be expressed by Equation 6 below.

[Equation 6]

$$eL(u) = e_1 * L_1 + \ldots + e_q * L_q$$

wherein

u is an integer of 1 or more, preferably an integer ranging from 1 to 6,

$e_1$ to $e_q$ are each independently a rational number of 0.08 to 0.65, and

$L_1$ to $L_q$ may each independently be the normalized expression level of any gene belonging to the fifth gene group.

In addition, as described herein, fM(v) may be expressed by Equation 7 below.

[Equation 7]

$$fM(v) = f_1 * M_1 + \ldots + f_q * M_q$$

wherein

v is an integer of 1 or more, preferably an integer ranging from 1 to 3,

$f_1$ to $f_q$ are each independently a rational number of -0.58 to -0.02, and

$M_1$ to $M_q$ may each independently be the normalized expression level of any gene belonging to the sixth gene group.

According to the present invention, the decision index (DI) is expressed by the Equation below:

Decision Index (DI) = 0.62 x KIF23 + 0.60 x SLC7A5 + 0.59 x KPNA2 + 0.53 x AURKA + 0.34 x E2F8 + 0.34 x MMP11 + 0.24 x SHCBP1 + 0.20 x CTSL2 + 0.16 x CENPE + 0.16 x TFRC + 0.15 x KIF 18A + 0.14 x CCNE2 + 0.04 x KIF 14 + 0.04 x RRM2 + (-0.04) x CX3CR1 + (-0.05) x JMJD5 + (-0.33) x CACNA1D + (-0.36) x ESR1 + (-0.48) x GSTM1 + (-1.45) x PGR + (-2.04) x SCUBE2 + 41.16.

In the present disclosure, the method further includes a step of determining that the prognosis of breast cancer is poor when the value of the calculated decision index (DI) is greater than 20.

The method for providing information on breast cancer prognosis according to the present disclosure is capable of predicting the prognosis of breast cancer in the subject, but also may be used for the decision of whether or not to use chemotherapy for the subject, prediction of treatment responsiveness of the subject to chemotherapy, or prediction of prognosis in the subject after anticancer chemotherapy.

Also described herein is a composition for predicting cancer prognosis containing an agent for measuring the expression level of each of a first gene group, a second gene group, a third gene group, a fourth gene group, a fifth gene group and a sixth gene group in a biological sample obtained from a subject.

As described herein, the first gene group may be any one or more selected from the group consisting of ESR1 (estrogen receptor 1), PGR (progesterone receptor), and SCUBE2 (signal peptide, CUB domain and EGF like domain containing 2).

As described herein, the second gene group may be any one or more selected from among CTSL2 (cathepsin V), and MMP11 (matrix metallopeptidase 11).

As described herein, the third gene group may be any one or more selected from among TFRC (transferrin receptor), and CX3CR1 (C-X3-C motif chemokine receptor 1).

As described herein, the fourth gene group may be any one or more selected from the group consisting of KIF14 (kinesin family member 14), RRM2 (ribonucleotide reductase regulatory subunit M2), SHCBP1 (SHC binding and spindle associated 1), SLC7A5 (solute carrier family 7 member 5), and KPNA2 (karyopherin subunit alpha 2).

As described herein, the fifth gene group may be any one or more selected from the group consisting of AURKA (aurora kinase A), CCNE2 (cyclin E2), CENPE (centromere protein E), E2F8 (E2F transcription factor 8), KIF18A (kinesin family member 18A), and KIF23 (kinesin family member 23).

As described herein, the sixth gene group may be any one or more selected from the group consisting of JMJD5 (lysine demethylase 8), CACNA1D (calcium voltage-gated channel subunit alpha1 D), and GSTM1 (glutathione S-transferase Mu 1).

The agent for measuring the expression level of the gene may be an antisense oligonucleotide, primer or probe capable of specifically binding to a gene of each of the first gene group, the second gene group, the third gene group, the fourth gene group, the fifth gene group and the sixth gene group, but is not limited thereto.

The composition for predicting cancer prognosis, as described herein, may further contain an agent for measuring the expression level of a reference gene to normalize the expression level of each of the first gene group, the second gene group, the third gene group, the fourth gene group, the fifth gene group and the sixth gene group.

Although the type of the reference gene is not particularly limited, but the reference gene may be, for example, any one or more selected from the group consisting of ACTB, APOBEC3B, ASF1B, ASPM, AURKB, BAG1, BCL2, BIRC5, BLM, BUB1, BUB1B, C14orf45, C16orf61, C7orf63, CCNA2, CCNB1, CCNB2, CCNE1, CCT5, CD68, CDC20, CDC25A, CDC45, CDC6, CDCA3, CDCA8, CDK1, CDKN3, CENPA, CENPF, CENPM, CENPN, CEP55, CHEK1, CIRBP, CKS2, CRIM1, CYBRD1, DBF4, DDX39, DLGAP5, DNMT3B, DONSON, DTL, E2F1, ECHDC2, ERBB2, ERCC6L, ESPL1, EXO1, EZH2, FAM64A, FANCI, FBXO5, FEN1, FOXM1, GAPDH, GINS1, GRB7, GTSE1, GUSB, HJURP, HMMR, HN1, IFT46, KIF11, KIF15, KIF18B, KIF20A, KIF2C, KIF4A, KIFC1, LMNB1, LMNB2, LRIG1, LRRC48, LRRC59, MAD2L1, MARCH8, MCM10, MCM2, MCM6, MELK, MKI67, MLF1IP, MYBL2, NCAPG, NCAPG2, NCAPH, NDC80, NEK2, NUP93, NUSAP1, OIP5, PBK, PDSS1, PKMYT1, PLK1, PLK4, PRC1, PTTG1, RACGAP1, RAD51, RAD51AP1, RAI2, RFC4, RPLPO, SETBP1, SF3B3, SHMT2, SLC25A12, SPAG5, SPC25, SQLE, STARD13, STIL, STMN1, SYNC, TACC3, TK1, TOP2A, TPX2, TRIP13, TROAP, TTK, UBE2C, UBE2S, ZWINT, CIOorf76, C12orf72, CIAO1, CNOT4, DBR1, DND1, FBXO42, GRK4, HNRNPK, HNRNPL, HNRNPR, KHDRBS1, KLRAQ1, LACE1, LOC148189, LOC285033, LOC493754, MRPL44, NRF1, PKNOX1, PPHLN1, RRN3P3, SENP8, SLC4A1AP, TARDBP, THRAP3, TTLL11, WDR33, and ZNF143.

The composition for predicting cancer prognosis, as described herein, is not only capable of predicting the prognosis of cancer in the subject, but also may be used for the decision of whether or not to use chemotherapy for the subject, prediction of treatment responsiveness of the subject to chemotherapy, or prediction of prognosis in the subject after anticancer chemotherapy.

According to the present disclosure, there is also provided a device for use in determining breast cancer prognosis including: (a) a detection unit configured to measure the expression levels of a first gene group, a second gene group, a third gene group, a fourth gene group, a fifth gene group and a sixth gene group in a biological sample obtained from a subject and normalize the measured expression levels; (b) an arithmetic unit configured to calculate a decision index (DI) by multiplying the expression level of each of the first gene group, the second gene group, the third gene group, the fourth gene group, the fifth gene group and the sixth gene group, normalized in the detection unit, by a regression coefficient (weight) value, and summing the multiplied values; and (c) an output unit configured to predict breast cancer prognosis in the subject by the decision index obtained in the arithmetic unit and output the predicted result.

The detection unit may measure the expression levels of the first gene group, the second gene group, the third gene group, the fourth gene group, the fifth gene group and the sixth gene group in the biological sample obtained from the subject.

In the present disclosure, the first gene group consists of ESR1 (estrogen receptor 1), PGR (progesterone receptor), and SCUBE2 (signal peptide, CUB domain and EGF like domain containing 2).

In the present disclosure, the second gene group consists of CTSL2 (cathepsin V), and MMP11 (matrix metallopeptidase 11).

In the present disclosure, the third gene group consists of TFRC (transferrin receptor), and CX3CR1 (C-X3-C motif chemokine receptor 1).

In the present disclosure, the fourth gene group consists of KIF14 (kinesin family member 14), RRM2 (ribonucleotide reductase regulatory subunit M2), SHCBP1 (SHC binding and spindle associated 1), SLC7A5 (solute carrier family 7 member 5), and KPNA2 (karyopherin subunit alpha 2).

In the present disclosure, the fifth gene group consists of AURKA (aurora kinase A), CCNE2 (cyclin E2), CENPE (centromere protein E), E2F8 (E2F transcription factor 8), KIF18A (kinesin family member 18A), and KIF23 (kinesin family member 23).

In the present disclosure, the sixth gene group consists of JMJD5 (lysine demethylase 8), CACNA1D (calcium voltage-gated channel subunit alpha1 D), and GSTM1 (glutathione S-transferase Mu 1).

In the present disclosure, the detection unit may normalize the measured expression levels of the first gene group, the second gene group, the third gene group, the fourth gene group, the fifth gene group and the sixth gene group to the expression level of the reference gene.

Although the type of the reference gene in the present disclosure is not particularly limited, but the reference gene may be, for example, any one or more selected from the group consisting of ACTB, APOBEC3B, ASF1B, ASPM, AURKB, BAG1, BCL2, BIRC5, BLM, BUB1, BUB1B, C14orf45, C16orf61, C7orf63, CCNA2, CCNB1, CCNB2, CCNE1, CCT5, CD68, CDC20, CDC25A, CDC45, CDC6, CDCA3, CDCA8, CDK1, CDKN3, CENPA, CENPF, CENPM, CENPN, CEP55, CHEK1, CIRBP, CKS2, CRIM1, CYBRD1, DBF4, DDX39, DLGAP5, DNMT3B, DONSON, DTL, E2F1, ECHDC2, ERBB2, ERCC6L, ESPL1, EXO1, EZH2, FAM64A, FANCI, FBXO5, FEN1, FOXM1, GAPDH, GINS1, GRB7, GTSE1, GUSB, HJURP, HMMR, HN1, IFT46, KIF11, KIF15, KIF18B, KIF20A, KIF2C, KIF4A, KIFC1, LMNB1, LMNB2, LRIG1, LRRC48, LRRC59, MAD2L1, MARCH8, MCM10, MCM2, MCM6, MELK, MKI67, MLF1IP, MYBL2, NCAPG, NCAPG2, NCAPH, NDC80, NEK2, NUP93, NUSAP1, OIP5, PBK, PDSS1, PKMYT1, PLK1, PLK4, PRC1, PTTG1, RACGAP1, RAD51, RAD51AP1, RAI2, RFC4, RPLPO, SETBP1, SF3B3, SHMT2, SLC25A12, SPAG5, SPC25, SQLE, STARD13, STIL, STMN1, SYNC, TACC3, TK1, TOP2A, TPX2, TRIP13, TROAP, TTK, UBE2C, UBE2S, ZWINT, C10orf76, C12orf72, CIAO1, CNOT4, DBR1, DND1, FBXO42, GRK4, HNRNPK, HNRNPL, HNRNPR, KHDRBS1, KLRAQ1, LACE1, LOC148189, LOC285033, LOC493754, MRPL44, NRF1, PKNOX1, PPHLN1, RRN3P3, SENP8, SLC4A1AP, TARDBP, THRAP3, TTLL11, WDR33, and ZNF143.

An agent, which is used to measure the expression level of the first gene group, the second gene group, the third gene group, the fourth gene group, the fifth gene group, the sixth gene group or the expression level of the reference gene in the detection unit of the present disclosure, and methods for measuring and normalizing the expression levels, overlap with those described above with respect to the method for providing information on cancer prognosis according to the present disclosure, and thus detailed description thereof will be omitted.

In the present disclosure, after the expression level of each of the first gene group, the second gene group, the third gene group, the fourth gene group, the fifth gene group and the sixth gene group is normalized in the detection unit, the decision index (DI) is calculated in the arithmetic unit by multiplying the expression level of each of the first gene group, the second gene group, the third gene group, the fourth gene group, the fifth gene group and the sixth gene group by the regression coefficient (weight) value and summing the multiplied values.

In the present disclosure, as the first gene group, the second gene group, the third gene group, the fourth gene group, the fifth gene group, or the sixth gene group includes a plurality of genes, the normalized expression level of each of the plurality of genes belonging to each gene group are multiplied by a regression coefficient value. In this case, even if the plurality of genes belong to the same gene group, the regression coefficient values multiplied by the respective normalized expression levels may be the same as or different from each other.

In the present disclosure, the decision index (DI) is obtained by multiplying the normalized expression level of each of the first gene group, the second gene group, the third gene group, the fourth gene group, the fifth gene group and the sixth gene group by the regression coefficient value and then summing the multiplied values. A correction coefficient value is additionally added.

As described herein, the correction coefficient value may be a rational number ranging from 35.09 to 47.07. According to the present invention, the correction coefficient value is 41.16.

As described herein, the decision index (DI) may be expressed as follows:

[Equation 1]

$$DI = aX(q) + bY(r) + cZ(s) + dK(t) + eL(u) + fM(v)$$

wherein

q, r, s, t, u and v are each independently an integer of 1 or more, preferably an integer ranging from 1 to 6. More preferably, q may be an integer ranging from 1 to 3, r may be an integer ranging from 1 to 2, s may be an integer ranging from 1 to 2, t may be an integer ranging from 1 to 5, u may be an integer ranging from 1 to 6, and v may be an integer ranging from 1 to 3.

a is a regression coefficient value multiplied by the normalized expression level of the first gene group, and may be a rational number ranging from -2.36 to -0.34.

b is a regression coefficient value multiplied by the normalized expression level of the second gene group, and may be a rational number ranging from 0.17 to 0.42.

c is a regression coefficient value multiplied by the normalized expression level of the third gene group, and may be a rational number ranging from -0.06 to 0.22.

d is a regression coefficient value multiplied by the normalized expression level of the fourth gene group, and may be a rational number ranging from 0.01 to 0.73.

e is a regression coefficient value multiplied by the normalized expression level of the fifth gene group, and may be a rational number ranging from 0.08 to 0.65.

f is a regression coefficient value multiplied by the normalized expression level of the fifth gene group, and may be a rational number ranging from -0.58 to -0.02.

X(q) may be the sum of the normalized expression levels of q genes belonging to the first gene group. However, when q is 1, X(q) may be a value of the normalized expression level of any one gene belonging to the first gene group. In addition, aX(q) may be the sum of the values obtained by multiplying the normalized expression level of each of q genes belonging to the first gene group by a, and when q is an integer of 2 or more, a values multiplied by the normalized expression level of each gene may be the same as or different from each other.

Y(r) may be the sum of the normalized expression levels of r genes belonging to the second gene group. However, when r is 1, Y(r) may be a value of the normalized expression level of any one gene belonging to the second gene group. In addition, bY(r) may be the sum of the values obtained by multiplying the normalized expression level of each of r genes belonging to the second gene group by b, and when r is an integer of 2 or more, b values multiplied by the normalized expression level of each gene may be the same as or different from each other.

Z(s) may be the sum of the normalized expression levels of s genes belonging to the third gene group. However, when s is 1, Z(s) may be a value of the normalized expression level of any one gene belonging to the third gene group. In addition, cZ(s) may be the sum of the values obtained by multiplying the normalized expression level of each of s genes belonging to the third gene group by c, and when s is an integer of 2 or more, c values multiplied by the normalized expression level of each gene may be the same as or different from each other.

K(t) may be the sum of the normalized expression levels of t genes belonging to the fourth gene group. However, when t is 1, K(t) may be a value of the normalized expression level of any one gene belonging to the fourth gene group. In addition, dK(t) may be the sum of the values obtained by multiplying the normalized expression level of each of t genes belonging to the fourth gene group by d, and when t is an integer of 2 or more, d values multiplied by the normalized expression level of each gene may be the same as or different from each other.

L(u) may be the sum of the normalized expression levels of u genes belonging to the fifth gene group. However, when u is 1, L(u) may be a value of the normalized expression level of any one gene belonging to the fifth gene group. In addition, the eL (u) may be the sum of values obtained by multiplying the normalized expression level of each of u genes belonging to the fifth gene group by e, and when u is an integer of 2 or more, e values multiplied by the normalized expression level of each gene may be the same as or different from each other.

M(v) may be the sum of the normalized expression levels of v genes belonging to the sixth gene group. However, when v is 1, M(v) may be a value of the normalized expression level of any one gene belonging to the sixth gene group. In addition, fM(v) may be the sum of values obtained by multiplying the normalized expression level of each of v genes belonging to the sixth gene group by f, and when v is an integer of 2 or more, f values multiplied by the normalized expression level of each gene may be the same or different from each other.

More specifically, aX(q) may be expressed by Equation 2 below.

$$[\text{Equation 2}]$$

$$aX(q) = a_1 * X_1 + \ldots + a_q * X_q$$

wherein

q is an integer of 1 or more, preferably an integer ranging from 1 to 3,
$a_1$ to $a_q$ are each independently a rational number ranging from -2.36 to -0.34,
$X_1$ to $X_q$ may each independently be the normalized expression level of any gene belonging to the first gene group.
Also, as described herein, bY(r) may be expressed by Equation 3 below.

$$[\text{Equation 3}]$$

$$bY(r) = b_1 * Y_1 + \ldots + b_q * Y_q$$

wherein

r is an integer of 1 or more, preferably an integer ranging from 1 to 2,
$b_1$ to $b_q$ are each independently a rational number ranging from 0.17 to 0.42,
$Y_1$ to $Y_q$ may each independently be the normalized expression level of any gene belonging to the second gene group.

In addition, as described herein, cZ(s) may be expressed by Equation 4 below.

$$[\text{Equation 4}]$$

$$cZ(s) = c_1 * Z_1 + \ldots + c_q * Z_q$$

wherein

13

s is an integer of 1 or more, preferably an integer ranging from 1 to 2,
$c_1$ to $c_q$ are each independently a rational number ranging from -0.06 to 0.22, and
$Z_1$ to $Z_q$ may each independently be the normalized expression level of any gene belonging to the third gene group.

In addition, as described herein, the dK(t) may be expressed by Equation 5 below.

$$[\text{Equation 5}]$$

$$dK(t) = d_1 * K_1 + \ldots + d_q * K_q$$

wherein

t is an integer of 1 or more, preferably an integer ranging from 1 to 5,
$d_i$ to $d_q$ are each independently a rational number of 0.01 to 0.73, and
$K_1$ to $K_q$ may each independently be the normalized expression level of any gene belonging to the fourth gene group.

In addition, as described herein, eL(u) may be expressed by Equation 6 below.

$$[\text{Equation 6}]$$

$$eL(u) = e_1 * L_1 + \ldots + e_q * L_q$$

wherein

u is an integer of 1 or more, preferably an integer ranging from 1 to 6,
$e_1$ to $e_q$ are each independently a rational number of 0.08 to 0.65, and
$L_1$ to $L_q$ may each independently be the normalized expression level of any gene belonging to the fifth gene group.

In addition, as described herein, fM(v) may be expressed by Equation 7 below.

$$[\text{Equation 7}]$$

$$fM(v) = f_1 * M_1 + \ldots + f_q * M_q$$

wherein

v is an integer of 1 or more, preferably an integer ranging from 1 to 3,
$f_1$ to $f_q$ are each independently a rational number of -0.58 to -0.02, and
$M_1$ to $M_q$ may each independently be the normalized expression level of any gene belonging to the sixth gene group.

According to the present invention, the decision index (DI) is expressed by the Equation below:

Decision Index (DI) = 0.62 x KIF23 + 0.60 x SLC7A5 + 0.59 x KPNA2 + 0.53 x AURKA + 0.34 x E2F8 + 0.34 x MMP11 + 0.24 x SHCBP1 + 0.20 x CTSL2 + 0.16 x CENPE + 0.16 x TFRC + 0.15 x KIF18A + 0.14 x CCNE2 + 0.04 x KIF14 + 0.04 x RRM2 + (-0.04) x CX3CR1 + (-0.05) x JMJD5 + (-0.33) x CACNA1D + (-0.36) x ESR1 + (-0.48) x GSTM1 + (-1.45) x PGR + (-2.04) x SCUBE2 + 41.16.

The output unit predicts and outputs the prognosis of breast cancer in the subject by the decision index (DI) obtained by the arithmetic unit, and predicts and outputs poor prognosis when the value of the decision index (DI) is greater than 20.
The device for use in determining breast cancer prognosis according to the present disclosure is capable of predicting the prognosis of breast cancer in the subject, but also may be used for the decision of whether or not to use chemotherapy for the subject, prediction of treatment responsiveness of the subject to chemotherapy, or prediction of prognosis in the subject after anticancer chemotherapy.

Advantageous Effects of Invention

According to the present disclosure, it is possible to predict breast cancer prognosis in all age groups ranging from 20s to 80s, and in particular, it is possible to predict breast cancer prognosis in women of both younger and older than 50 years of age. In addition, according to the present disclosure, it is possible to predict whether a breast cancer patient is more likely respond favorably to treatment in chemotherapy.

Brief Description of Drawings

FIGS. 1a and 1b are graphs showing the results of predicting prognosis of breast cancer by the method for providing information on cancer prognosis according to the present disclosure, in comparison with the results obtained by a conventional product.

FIG. 2 is a graph confirming the hazard ratio when the method for providing information on the prognosis of cancer according to the present disclosure was performed.

FIGS. 3a and 3b are graphs showing the results of comparing patients aged 50 years or younger with patients over 50 years of age according to the method for providing information on cancer prognosis according to the present disclosure.

Best Mode

The present invention is directed to a method for providing information on breast cancer prognosis, the method including steps of: (a) measuring the expression levels of a first gene group, a second gene group, a third gene group, a fourth gene group, a fifth gene group and a sixth gene group in a biological sample obtained from a subject, and normalizing the measured expression levels; and (b) calculating a decision index (DI) by multiplying the expression level of each of the first gene group, the second gene group, the third gene group, the fourth gene group, the fifth gene group and the sixth gene group, normalized in step (a), by a regression coefficient value, and summing the multiplied values, thereby predicting breast cancer prognosis in the subject.

Also described herein is a composition for predicting cancer prognosis, the composition containing an agent for measuring the expression level of each of a first gene group, a second gene group, a third gene group, a fourth gene group, a fifth gene group and a sixth gene group in a biological sample obtained from a subject.

Still further the present invention is directed to a device for use in determining breast cancer prognosis including: (a) a detection unit configured to measure the expression levels of a first gene group, a second gene group, a third gene group, a fourth gene group, a fifth gene group and a sixth gene group in a biological sample obtained from a subject and normalize the measured expression levels; (b) an arithmetic unit configured to calculate a decision index (DI) by multiplying the expression level of each of the first gene group, the second gene group, the third gene group, the fourth gene group, the fifth gene group and the sixth gene group, normalized in the detection unit, by a regression coefficient (weight) value, and summing the multiplied values; and (c) an output unit configured to predict cancer prognosis in the subject by the decision index obtained in the arithmetic unit and output the predicted result.

In the present disclosure, the first gene group consists of ESR1 (estrogen receptor 1), PGR (progesterone receptor), and SCUBE2 (signal peptide, CUB domain and EGF like domain containing 2).

In the present disclosure, the second gene group consists of CTSL2 (cathepsin V), and MMP11 (matrix metallopeptidase 11).

In the present disclosure, the third gene group consists of TFRC (transferrin receptor), and CX3CR1 (C-X3-C motif chemokine receptor 1).

In the present disclosure, the fourth gene group consists of KIF14 (kinesin family member 14), RRM2 (ribonucleotide reductase regulatory subunit M2), SHCBP1 (SHC binding and spindle associated 1), SLC7A5 (solute carrier family 7 member 5), and KPNA2 (karyopherin subunit alpha 2).

In the present disclosure, the fifth gene group consists of AURKA (aurora kinase A), CCNE2 (cyclin E2), CENPE (centromere protein E), E2F8 (E2F transcription factor 8), KIF18A (kinesin family member 18A), and KIF23 (kinesin family member 23).

In the present disclosure, the sixth gene group consists of JMJD5 (lysine demethylase 8), CACNA1D (calcium voltage-gated channel subunit alpha1 D), and GSTM1 (glutathione S-transferase Mu 1).

Mode for Invention

Hereinafter, the present disclosure will be described in more detail with reference to examples. It will be obvious to those skilled in the art that these examples are only for explaining the present disclosure in more detail, and the scope of the present disclosure according to the subject matter of the present disclosure is not limited by these examples.

## Examples

Selection of Subject Breast Cancer Patients and Preparation of Test Tissue

From hormone receptor-positive, lymph node metastasis-negative stage 1-2 breast cancer surgical tissues, representative formalin-fixed paraffin-embedded (FFPE) blocks were selected and RNAs were extracted therefrom.

## Target RNA Sequencing

When RNAs had a certain level of quality, a cDNA library was made therefrom. Only specific genes were detected using 179 gene panel probes. All of the 179 genes were sequenced using a next generation sequencing (NGS) system and aligned to the human genome. The reads were aligned to the public human genome reference using the alignment algorithm STAR. The expression level of each gene was measured from information on the aligned analysis sequences.

## Normalization of Targeted RNA-seq Expression Information

Normalization was performed using patients used for modeling and the genes of the patients. Normalization was performed using 343 patients and 179 genes. Only 21 genes were used to calculate the recurrence score, and all genes were used for normalization.

The present disclosure has provided genes and gene sets useful for predicting the response of cancer (e.g., breast cancer) patients to chemotherapy. The present disclosure has also provided a clinically effective test that uses multi-gene RNA analysis to predict the response of breast cancer patients to chemotherapy.

The present inventors have selected and identified a set of genes useful for predicting whether cancer patients, e.g., breast cancer patients, are more likely to respond favorably to chemotherapy. For the prediction as described above, the following six gene groups were categorized and selected: (1) an estrogen-related gene group, (2) an invasion-related gene group, (3) an immune-related gene group, (4) a proliferation-related gene group, (5) a cell cycle-related gene group, and (6) other gene group. More specifically, the genes selected by the present inventors were selected from the group consisting of ESR1 (estrogen receptor 1), PGR (progesterone receptor), SCUBE2 (signal peptide, CUB domain and EGF like domain containing 2), CTSL2 (cathepsin V), MMP11 (matrix metallopeptidase 11), TFRC (transferrin receptor), CX3CR1 (C-X3-C motif chemokine receptor 1), KIF14 (kinesin family member 14), RRM2 (ribonucleotide reductase regulatory subunit M2), SHCBP1 (SHC binding and spindle associated 1), SLC7A5 (solute carrier family 7 member 5), KPNA2 (karyopherin subunit alpha 2), AURKA (aurora kinase A), CCNE2 (cyclin E2), CENPE (centromere protein E), E2F8 (E2F transcription factor 8), KIF18A (kinesin family member 18A), KIF23 (kinesin family member 23), JMJD5 (lysine demethylase 8), CACNA1D (calcium voltage-gated channel subunit alpha1 D), and GSTM1 (glutathione S-transferase Mu 1).

## Algorithm for Prediction of Breast Cancer Prognosis and Prediction of Treatment Effect of Chemotherapy

Decision Index (DI) values were calculated using regression coefficient (weight) values and a correction coefficient for the 21 genes obtained from the predictive model. Here, the predictive model was a model constructed by Lasso regression in the Algorithm building process to estimate the decision index values for 250 patients, and subjected to validation with 93 persons and then to clinical verification with 413 persons.

The 21 genes were categorized into: (1) an estrogen-related first gene group consisting of ESR1 (estrogen receptor 1), PGR (progesterone receptor), and SCUBE2 (signal peptide, CUB domain and EGF like domain containing 2); (2) an invasion-related second gene group consisting of CTSL2 (cathepsin V), and MMP11 (matrix metallopeptidase 11); (3) an immune-related third gene group consisting of TFRC (transferrin receptor), and CX3CR1 (C-X3-C motif chemokine receptor 1); (4) a proliferation-related fourth gene group consisting of KIF14 (kinesin family member 14), RRM2 (ribonucleotide reductase regulatory subunit M2), SHCBP1 (SHC binding and spindle associated 1), SLC7A5 (solute carrier family 7 member 5), and KPNA2 (karyopherin subunit alpha 2); (5) a cell cycle-related fifth gene group consisting of AURKA (aurora kinase A), CCNE2 (cyclin E2), CENPE (centromere protein E), E2F8 (E2F transcription factor 8), KIF18A (kinesin family member 18A), and KIF23 (kinesin family member 23), and (6) a six gene group consisting of JMJD5 (lysine demethylase 8), CACNA1D (calcium voltage-gated channel subunit alpha1 D), and GSTM1 (glutathione S-transferase Mu 1).

It was confirmed that a suitable range of the regression coefficient (weight) value for each of the six gene group was a rational number ranging from -2.36 to -0.34 for the estrogen-related first gene group, a rational number ranging from 0.17 to 0.42 for the invasion-related second gene group, a rational number ranging from -0.06 to 0.22 for the immune-related third gene group, a rational number ranging from 0.01 to 0.73 for the proliferation-related fourth gene group, a rational number ranging from 0.08 to 0.65 for the cell cycle-related fifth gene group, and a rational number ranging from -0.58 to -0.02 for the sixth group, and a suitable range of the correction coefficient value was a rational number ranging from 35.09 to 47.07.

The decision index (DI) value was calculated as follows.

Decision Index (DI) = 0.62 x KIF23 + 0.60 x SLC7A5 + 0.59 x KPNA2 + 0.53 x AURKA + 0.34 x E2F8 + 0.34 x MMP11 + 0.24 x SHCBP1 + 0.20 x CTSL2 + 0.16 x CENPE + 0.16 x TFRC + 0.15 x KIF 18A + 0.14 x CCNE2 + 0.04 x KIF 14 + 0.04 x RRM2 + (-0.04) x CX3CR1 + (-0.05) x JMJD5 + (-0.33) x CACNA1D + (-0.36) x ESR1 + (-0.48) x GSTM1 + (-1.45) x PGR + (-2.04) x SCUBE2 + 41.16. Using the calculated DI value, patient's prognosis prediction and diagnosis was performed.

In the present disclosure, it was confirmed that, through the prognostic score values as described above, it was possible to diagnose breast cancer in all age groups, particularly in an age group ranging from 20s to 80s in one embodiment, and in pre- and postmenopausal women in another embodiment, and in particular, it is possible to diagnose breast cancer in early stage breast cancer patients. It was confirmed that the composition and method according to the present disclosure could diagnose ER+/HER2-breast cancer in all age groups, particularly in an age group ranging from 20s to 80s in one embodiment, and in pre- and postmenopausal women in another embodiment.

## Validation of Prediction of Breast Cancer Prognosis

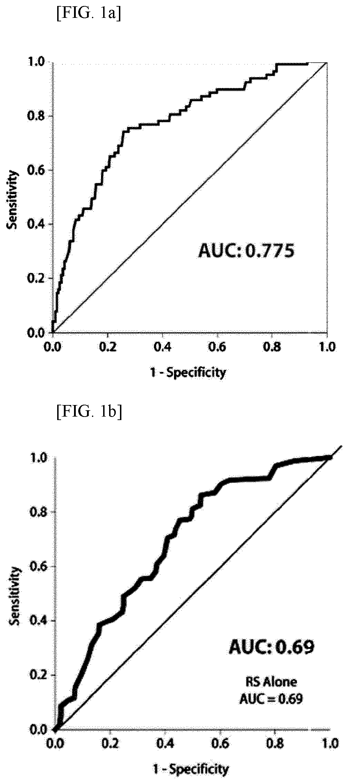

Clinical validation was conducted for 413 patients who have been followed up for 5 years or more, among ER+/HER2- breast cancer patients without lymph node metastasis. Decision index (DI) values for all the patients were calculated using the prognostic predictive tool specified above. In order to check any relationship between sensitivity and specificity at all cut-offs, a ROC curve (Receiver-Operating Characteristic curve) was drawn, and an AUC (Area under Curve) for the ROC curve was calculated and compared with the value obtained by a commercial product. It was confirmed that the accuracy of the result of predicting breast cancer prognosis by the method for providing information on cancer prognosis according to the present disclosure (FIG. 1(a)) was superior to that of a commercially available product (Oncotype Dx FIG. 1(b)) (see FIG. 1).

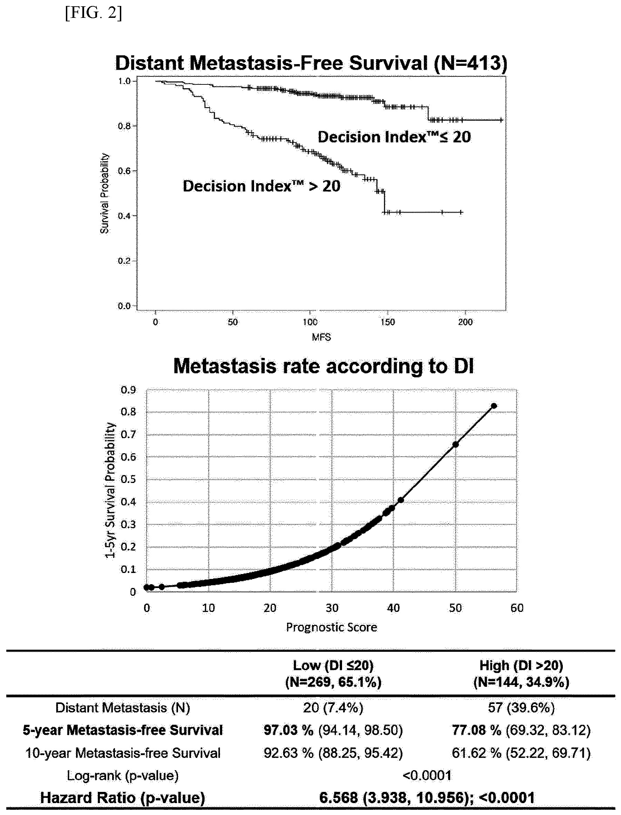

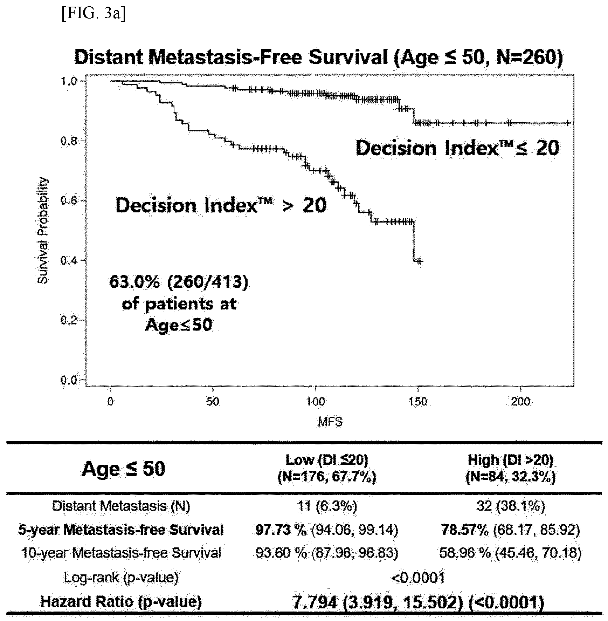

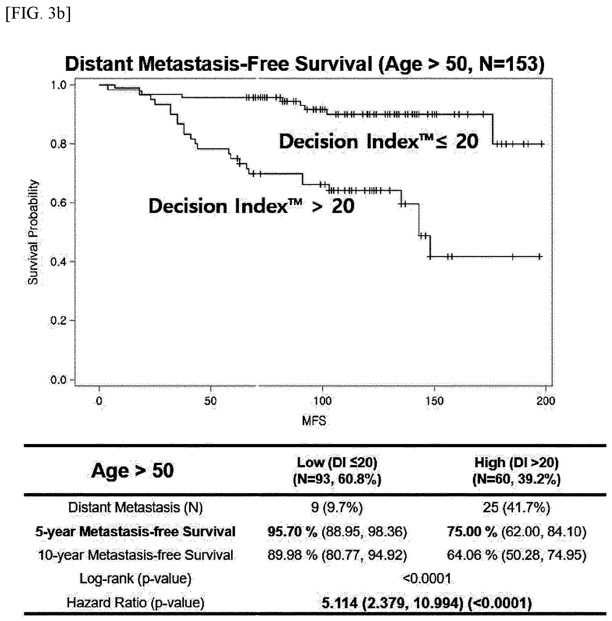

Considering the relationship between the decision index (DI) and the hazard ratio (HR), an optimal cut-off was set and survival analysis about the development of distant metastasis was performed. As a result, it could be confirmed that, when the method for providing information on cancer prognosis according to the present disclosure was performed on all the patients, it showed a hazard ratio of about 6.6, suggesting that it has excellent predictive power (see FIG. 2). Using the method for providing information on cancer prognosis according to the present disclosure, survival analysis about the development of distant metastasis was performed on patients less than or equal to 50 years of age (FIG. 3(a)) and patients over 50 years of age (FIG. 3(b)). It could be confirmed that the present disclosure exhibited excellent performance consistently regardless of age criteria around the age of 50 (see FIG. 3).

## Industrial Applicability

The present disclosure provides useful gene expression information for predicting whether breast cancer patients are more likely to respond favorably to treatment in chemotherapy. According to the present disclosure, it is possible to predict breast cancer prognosis in all age groups ranging from 20s to 80s, and in particular, it is possible to predict breast cancer prognosis in women of both younger and older than 50 years of age.

## Claims

**1.** A method for determining breast cancer prognosis, the method comprising steps of:

(a) measuring expression levels of a first gene group, a second gene group, a third gene group, a fourth gene group, a fifth gene group and a sixth gene group in a biological sample obtained from a subject, and normalizing the measured expression levels;
(b) calculating a decision index (DI) by multiplying the expression level of each of the first gene group, the second gene group, the third gene group, the fourth gene group, the fifth gene group and the sixth gene group, normalized in step (a), by a regression coefficient (weight) value, and summing the multiplied values, thereby predicting breast cancer prognosis in the subject, and
(c) determining that the prognosis of breast cancer is poor when the value of the decision index (DI) is greater than 20,

wherein:

the first gene group consists of ESR1 (estrogen receptor 1), PGR (progesterone receptor), and SCUBE2 (signal peptide, CUB domain and EGF like domain containing 2);

the second gene group consists of CTSL2 (cathepsin V), and MMP11 (matrix metallopeptidase 11);

the third gene group consists of TFRC (transferrin receptor), and CX3CR1 (C-X3-C motif chemokine receptor 1);

the fourth gene group consists of KIF14 (kinesin family member 14), RRM2 (ribonucleotide reductase regulatory subunit M2), SHCBP1 (SHC binding and spindle associated 1), SLC7A5 (solute carrier family 7 member 5), and KPNA2 (karyopherin subunit alpha 2);

the fifth gene group consists of AURKA (aurora kinase A), CCNE2 (cyclin E2), CENPE (centromere protein E), E2F8 (E2F transcription factor 8), KIF18A (kinesin family member 18A), and KIF23 (kinesin family member 23); and

the sixth gene group consists of JMJD5 (lysine demethylase 8), CACNA1D (calcium voltage-gated channel subunit alpha1 D), and GSTM1 (glutathione S-transferase Mu 1),

wherein the decision index (DI) is expressed by the equation below:

$$DI = 0.62 \times KIF23 + 0.60 \times SLC7A5 + 0.59 \times KPNA2 + 0.53 \times AURKA + 0.34 \times E2F8 + 0.34 \times MMP11 + 0.24 \times SHCBP1 + 0.20 \times CTSL2 + 0.16 \times CENPE + 0.16 \times TFRC + 0.15 \times KIF18A + 0.14 \times CCNE2 + 0.04 \times KIF14 + 0.04 \times RRM2 + (-0.04) \times CX3CR1 + (-0.05) \times JMJD5 + (-0.33) \times CACNA1D + (-0.36) \times ESR1 + (-0.48) \times GSTM1 + (-1.45) \times PGR + (-2.04) \times SCUBE2 + 41.16$$

2. The method of claim 1, wherein the step of normalizing the measured expression levels comprises normalizing the expression level of each gene group using any one or more reference genes selected from the group consisting of ACTB, APOBEC3B, ASF1B, ASPM, AURKB, BAG1, BCL2, BIRC5, BLM, BUB1, BUB1B, C14orf45, C16orf61, C7orf63, CCNA2, CCNB1, CCNB2, CCNE1, CCT5, CD68, CDC20, CDC25A, CDC45, CDC6, CDCA3, CDCA8, CDK1, CDKN3, CENPA, CENPF, CENPM, CENPN, CEP55, CHEK1, CIRBP, CKS2, CRIM1, CYBRD1, DBF4, DDX39, DLGAP5, DNMT3B, DONSON, DTL, E2F1, ECHDC2, ERBB2, ERCC6L, ESPL1, EXO1, EZH2, FAM64A, FANCI, FBXO5, FEN1, FOXM1, GAPDH, GINS1, GRB7, GTSE1, GUSB, HJURP, HMMR, HN1, IFT46, KIF11, KIF15, KIF18B, KIF20A, KIF2C, KIF4A, KIFC1, LMNB1, LMNB2, LRIG1, LRRC48, LRRC59, MAD2L1, MARCH8, MCM10, MCM2, MCM6, MELK, MKI67, MLF1IP, MYBL2, NCAPG, NCAPG2, NCAPH, NDC80, NEK2, NUP93, NUSAP1, OIP5, PBK, PDSS1, PKMYT1, PLK1, PLK4, PRC1, PTTG1, RACGAP1, RAD51, RAD51AP1, RAI2, RFC4, RPLPO, SETBP1, SF3B3, SHMT2, SLC25A12, SPAG5, SPC25, SQLE, STARD13, STIL, STMN1, SYNC, TACC3, TK1, TOP2A, TPX2, TRIP13, TROAP, TTK, UBE2C, UBE2S, ZWINT, C10orf76, C12orf72, CIAO1, CNOT4, DBR1, DND1, FBXO42, GRK4, HNRNPK, HNRNPL, HNRNPR, KHDRBS1, KLRAQ1, LACE1, LOC148189, LOC285033, LOC493754, MRPL44, NRF1, PKNOX1, PPHLN1, RRN3P3, SENP8, SLC4AIAP, TARDBP, THRAP3, TTLL11, WDR33, and ZNF143.

3. A device for use in determining breast cancer prognosis, the device comprising:

(a) a detection unit configured to measure expression levels of a first gene group, a second gene group, a third gene group, a fourth gene group, a fifth gene group and a sixth gene group in a biological sample obtained from a subject and normalize the measured expression levels;

(b) an arithmetic unit configured to calculate a decision index (DI) by multiplying the expression level of each of the first gene group, the second gene group, the third gene group, the fourth gene group, the fifth gene group and the sixth gene group, normalized in the detection unit, by a regression coefficient (weight) value, and summing the multiplied values; and

(c) an output unit configured to predict breast cancer prognosis in the subject by the decision index obtained in the arithmetic unit and output the predicted result,

wherein:

the first gene group consists of ESR1 (estrogen receptor 1), PGR (progesterone receptor), and SCUBE2 (signal peptide, CUB domain and EGF like domain containing 2);

the second gene group consists of CTSL2 (cathepsin V), and MMP11 (matrix metallopeptidase 11);

the third gene group consists of TFRC (transferrin receptor), and CX3CR1 (C-X3-C motif chemokine receptor 1);

the fourth gene group consists of KIF14 (kinesin family member 14), RRM2 (ribonucleotide reductase regulatory subunit M2), SHCBP1 (SHC binding and spindle associated 1), SLC7A5 (solute carrier family 7 member 5), and KPNA2 (karyopherin subunit alpha 2);

the fifth gene group consists of AURKA (aurora kinase A), CCNE2 (cyclin E2), CENPE (centromere protein E), E2F8 (E2F transcription factor 8), KIF18A (kinesin family member 18A), and KIF23 (kinesin family member 23); and

the sixth gene group consists of JMJD5 (lysine demethylase 8), CACNA1D (calcium voltage-gated channel subunit alpha1 D), and GSTM1 (glutathione S-transferase Mu 1),

wherein the decision index (DI) is expressed by the equation below:

DI = 0.62 x KIF23 + 0.60 x SLC7A5 + 0.59 x KPNA2 + 0.53 x AURKA + 0.34 x E2F8 + 0.34 x MMP11 + 0.24 x SHCBP1 + 0.20 x CTSL2 + 0.16 x CENPE + 0.16 x TFRC + 0.15 x KIF18A + 0.14 x CCNE2 + 0.04 x KIF14 + 0.04 x RRM2 + (-0.04) x CX3CR1 + (-0.05) x JMJD5 + (-0.33) x CACNA1D + (-0.36) x ESR1 + (-0.48) x GSTM1 + (-1.45) x PGR + (-2.04) x SCUBE2 + 41.16

wherein the output unit determines that the prognosis of breast cancer is poor when the value of the decision index (DI) is greater than 20.

**4.** The device of claim 3, wherein the detection unit normalizes the expression levels of the first gene group, the second gene group, the third gene group, the fourth gene group, the fifth gene group and the sixth gene group to an expression level of a reference gene, wherein the reference gene is any one or more selected from the group consisting of ACTB, APOBEC3B, ASF1B, ASPM, AURKB, BAG1, BCL2, BIRC5, BLM, BUB1, BUB1B, C14orf45, C16orf61, C7orf63, CCNA2, CCNB1, CCNB2, CCNE1, CCT5, CD68, CDC20, CDC25A, CDC45, CDC6, CDCA3, CDCA8, CDK1, CDKN3, CENPA, CENPF, CENPM, CENPN, CEP55, CHEK1, CIRBP, CKS2, CRIM1, CYBRD1, DBF4, DDX39, DLGAP5, DNMT3B, DONSON, DTL, E2F1, ECHDC2, ERBB2, ERCC6L, ESPL1, EXO1, EZH2, FAM64A, FANCI, FBXO5, FEN1, FOXM1, GAPDH, GINS1, GRB7, GTSE1, GUSB, HJURP, HMMR, HN1, IFT46, KIF11, KIF15, KIF18B, KIF20A, KIF2C, KIF4A, KIFC1, LMNB1, LMNB2, LRIG1, LRRC48, LRRC59, MAD2L1, MARCH8, MCM10, MCM2, MCM6, MELK, MKI67, MLF1IP, MYBL2, NCAPG, NCAPG2, NCAPH, NDC80, NEK2, NUP93, NUSAP1, OIP5, PBK, PDSS1, PKMYT1, PLK1, PLK4, PRC1, PTTG1, RACGAP1, RAD51, RAD51AP1, RAI2, RFC4, RPLP0, SETBP1, SF3B3, SHMT2, SLC25A12, SPAG5, SPC25, SQLE, STARD13, STIL, STMN1, SYNC, TACC3, TK1, TOP2A, TPX2, TRIP13, TROAP, TTK, UBE2C, UBE2S, ZWINT, C10orf76, C12orf72, CIAO1, CNOT4, DBR1, DND1, FBXO42, GRK4, HNRNPK, HNRNPL, HNRNPR, KHDRBS1, KLRAQ1, LACE1, LOC148189, LOC285033, LOC493754, MRPL44, NRF1, PKNOX1, PPHLN1, RRN3P3, SENP8, SLC4A1AP, TARDBP, THRAP3, TTLL11, WDR33, and ZNF143.

## Patentansprüche

**1.** Verfahren zum Bestimmen einer Brustkrebs-Prognose, wobei das Verfahren die Schritte umfasst:

(a) Messen von Expressionsniveaus einer ersten Gengruppe, einer zweiten Gengruppe, einer dritten Gengruppe, einer vierten Gengruppe, einer fünften Gengruppe und einer sechsten Gengruppe in einer von einem Lebewesen erhaltenen biologischen Probe und Normalisieren der gemessenen Expressionsniveaus;
(b) Berechnen eines Entscheidungsindex (DI) durch Multiplizieren des Expressionsniveaus jeder der ersten Gengruppe, der zweiten Gengruppe, der dritten Gengruppe, der vierten Gengruppe, der fünften Gengruppe und der sechsten Gengruppe, normalisiert in Schritt (a), mit einem Regressionskoeffizientenwert (Gewichtung) und Aufsummieren der multiplizierten Werte, wodurch eine Brustkrebs-Prognose bei dem Lebewesen prognostiziert wird, und
(c) Bestimmen, dass die Brustkrebs-Prognose schlecht ist, wenn der Wert des Entscheidungsindex (DI) größer als 20 ist,

wobei:

die erste Gengruppe aus ESR1 (Östrogen-Rezeptor 1), PGR (Progesteron-Rezeptor) und SCUBE2 (Signalpeptid, CUB-Domäne und EGF-artige Domäne enthaltendes Protein 2) besteht;
die zweite Gengruppe aus CTSL2 (Cathepsin V) und MMP11 (Matrix-Metallopeptidase 11) besteht;
die dritte Gengruppe aus TFRC (Transferrin-Rezeptor) und CX3CR1 (C-X3-C-Motiv-Che-mokin-Rezeptor 1) besteht;

die vierte Gengruppe aus KIF14 (Kinesin-Familienmitglied 14), RRM2 (Ribonucleotid-Reduktase-regulatorische Untereinheit M2), SHCBP1 (SHC-bindendes und Spindel-assoziiertes Protein 1), SLC7A5 (Solvat-Carrier-Familie 7-Mitglied 5) und KPNA2 (Karyopherin-Untereinheit alpha 2) besteht;

die fünfte Gengruppe aus AURKA (Aurorakinase A), CCNE2 (Cyclin E2), CENPE (Centromer-Protein E), E2F8 (E2F-Transkriptionsfaktor 8), KIF18A (Kinesin-Familienmitglied 18A) und KIF23 (Kinesin-Familienmitglied 23) besteht; und

die sechste Gengruppe aus JMJD5 (Lysin-Demethylase 8), CACNA1D (spannungsgesteuerter Calciumkanal-Untereinheit alpha1 D) und GSTM1 (Glutathion S-Transferase Mu 1) besteht,

wobei der Entscheidungsindex (DI) durch die nachstehende Gleichung ausgedrückt wird:

$$DI = 0{,}62 \times KIF23 + 0{,}60 \times SLC7A5 + 0{,}59 \times KPNA2 + 0{,}53 \times AURKA + 0{,}34 \times E2F8 + 0{,}34 \times MMP11 + 0{,}24 \times SHCBP1 + 0{,}20 \times CTSL2 + 0{,}16 \times CENPE + 0{,}16 \times TFRC + 0{,}15 \times KIF18A + 0{,}14 \times CCNE2 + 0{,}04 \times KIF14 + 0{,}04 \times RRM2 + (-0{,}04) \times CX3CR1 + (-0{,}05) \times JMJD5 + (-0{,}33) \times CACNA1D + (-0{,}36) \times ESR1 + (-0{,}48) \times GSTM1 + (-1{,}45) \times PGR + (-2{,}04) \times SCUBE2 + 41{,}16$$

2. Verfahren nach Anspruch 1, wobei der Schritt eines Normalisierens der gemessenen Expressionsniveaus ein Normalisieren des Expressionsniveaus jeder Gengruppe unter Verwendung eines jeglichen oder mehreren Referenzgenen umfasst, die aus der Gruppe ausgewählt sind, die aus ACTB, APOBEC3B, ASF1B, ASPM, AURKB, BAG1, BCL2, BIRC5, BLM, BUB1, BUB1B, C14orf45, C16orf61, C7orf63, CCNA2, CCNB1, CCNB2, CCNE1, CCT5, CD68, CDC20, CDC25A, CDC45, CDC6, CDCA3, CDCA8, CDK1, CDKN3, CENPA, CENPF, CENPM, CENPN, CEP55, CHEK1, CIRBP, CKS2, CRIM1, CYBRD1, DBF4, DDX39, DLGAP5, DNMT3B, DONSON, DTL, E2F1, ECHDC2, ERBB2, ERCC6L, ESPL1, EXO1, EZH2, FAM64A, FANCI, FBXO5, FEN1, FOXM1, GAPDH, GINS1, GRB7, GTSE1, GUSB, HJURP, HMMR, HN1, IFT46, KIF11, KIF15, KIF18B, KIF20A, KIF2C, KIF4A, KIFC1, LMNB1, LMNB2, LRIG1, LRRC48, LRRC59, MAD2L1, MARCH8, MCM10, MCM2, MCM6, MELK, MKI67, MLF1IP, MYBL2, NCAPG, NCAPG2, NCAPH, NDC80, NEK2, NUP93, NUSAP1, OIP5, PBK, PDSS1, PKMYT1, PLK1, PLK4, PRC1, PTTG1, RACGAP1, RAD51, RAD51AP1, RAI2, RFC4, RPLPO, SETBP1, SF3B3, SHMT2, SLC25A12, SPAG5, SPC25, SQLE, STARD13, STIL, STMN1, SYNC, TACC3, TK1, TOP2A, TPX2, TRIP13, TROAP, TTK, UBE2C, UBE2S, ZWINT, C10orf76, C12orf72, CIAO1, CNOT4, DBR1, DND1, FBXO42, GRK4, HNRNPK, HNRNPL, HNRNPR, KHDRBS1, KLRAQ1, LACE1, LOC148189, LOC285033, LOC493754, MRPL44, NRF1, PKNOX1, PPHLN1, RRN3P3, SENP8, SLC4A1AP, TARDBP, THRAP3, TTLL11, WDR33 und ZNF143 besteht.

3. Vorrichtung zur Verwendung beim Bestimmen einer Brustkrebs-Prognose, wobei die Vorrichtung umfasst:

(a) eine Nachweiseinheit, die konfiguriert ist, Expressionsniveaus einer ersten Gengruppe, einer zweiten Gengruppe, einer dritten Gengruppe, einer vierten Gengruppe, einer fünften Gengruppe und einer sechsten Gengruppe in einer von einem Lebewesen erhaltenen biologischen Probe zu messen und die gemessenen Expressionsniveaus zu normalisieren;

(b) eine arithmetische Einheit, die konfiguriert ist, einen Entscheidungsindex (DI) dadurch zu berechnen, dass das Expressionsniveau jeder der ersten Gengruppe, der zweiten Gengruppe, der dritten Gengruppe, der vierten Gengruppe, der fünften Gengruppe und der sechsten Gengruppe, normalisiert in der Nachweiseinheit, mit einen Regressionskoeffizientenwert (Gewichtung) multipliziert wird und die multiplizierten Werte aufsummiert werden; und

(c) eine Ausgabeeinheit, die konfiguriert ist, eine Brustkrebs-Prognose bei dem Lebewesen durch den in der arithmetischen Einheit erhaltenen Entscheidungsindex zu prognostizieren und das prognostizierte Ergebnis auszugeben,

wobei:

die erste Gengruppe aus ESR1 (Östrogen-Rezeptor 1), PGR (Progesteron-Rezeptor) und SCUBE2 (Signalpeptid, CUB-Domäne und EGF-artige Domäne enthaltendes Protein 2) besteht;

die zweite Gengruppe aus CTSL2 (Cathepsin V) und MMP11 (Matrix-Metallopeptidase 11) besteht;

die dritte Gengruppe aus TFRC (Transferrin-Rezeptor) und CX3CR1 (C-X3-C-Motiv-Che-mokin-Rezeptor 1) besteht;

die vierte Gengruppe aus KIF14 (Kinesin-Familienmitglied 14), RRM2 (Ribonucleotid-Reduktase-regulatorische Untereinheit M2), SHCBP1 (SHC-bindendes und Spindel-assoziiertes Protein 1), SLC7A5 (Solvat-Carrier-Familie 7-Mitglied 5) und KPNA2 (Karyopherin-Untereinheit alpha 2) besteht;

die fünfte Gengruppe aus AURKA (Aurorakinase A), CCNE2 (Cyclin E2), CENPE (Centromer-Protein E), E2F8 (E2F-Transkriptionsfaktor 8), KIF18A (Kinesin-Familienmitglied 18A) und KIF23 (Kinesin-Familienmitglied 23) besteht; und

die sechste Gengruppe aus JMJD5 (Lysin-Demethylase 8), CACNA1D (spannungsgesteuerter Calciumkanal-Untereinheit alphal D) und GSTM1 (Glutathion S-Transferase Mu 1) besteht,

wobei der Entscheidungsindex (DI) durch die nachstehende Gleichung ausgedrückt wird:

DI = 0,62 x KIF23 + 0,60 x SLC7A5 + 0,59 x KPNA2 + 0,53 x AURKA + 0,34 x E2F8 + 0,34 x MMP11 + 0,24 x SHCBP1 + 0,20 x CTSL2 + 0,16 x CENPE + 0,16 x TFRC + 0,15 x KIF18A + 0,14 x CCNE2 + 0,04 x KIF14 + 0,04 x RRM2 + (-0,04) x CX3CR1 + (-0,05) x JMJD5 + (-0,33) x CACNA1D + (-0,36) x ESR1 + (-0,48) x GSTM1 + (-1,45) x PGR + (-2,04) x SCUBE2 + 41,16

wobei die Ausgabeeinheit bestimmt, das die Brustkrebs-Prognose schlecht ist, wenn der Wert des Entscheidungsindex (DI) größer als 20 ist.

4. Vorrichtung nach Anspruch 3, wobei die Nachweiseinheit die Expressionsniveaus der ersten Gengruppe, der zweiten Gengruppe, der dritten Gengruppe, der vierten Gengruppe, der fünften Gengruppe und der sechsten Gengruppe auf ein Expressionsniveau eines Referenzgens normalisiert, wobei das Referenzgen ein jegliches oder mehrere ist, die aus der Gruppe ausgewählt sind, die aus ACTB, APOBEC3B, ASF1B, ASPM, AURKB, BAG1, BCL2, BIRC5, BLM, BUB1, BUB1B, C14orf45, C16orf61, C7orf63, CCNA2, CCNB1, CCNB2, CCNE1, CCT5, CD68, CDC20, CDC25A, CDC45, CDC6, CDCA3, CDCA8, CDK1, CDKN3, CENPA, CENPF, CENPM, CENPN, CEP55, CHEK1, CIRBP, CKS2, CRIM1, CYBRD1, DBF4, DDX39, DLGAP5, DNMT3B, DONSON, DTL, E2F1, ECHDC2, ERBB2, ERCC6L, ESPL1, EXO1, EZH2, FAM64A, FANCI, FBXO5, FEN1, FOXM1, GAPDH, GINS1, GRB7, GTSE1, GUSB, HJURP, HMMR, HN1, IFT46, KIF11, KIF15, KIF18B, KIF20A, KIF2C, KIF4A, KIFC1, LMNB1, LMNB2, LRIG1, LRRC48, LRRC59, MAD2L1, MARCH8, MCM10, MCM2, MCM6, MELK, MKI67, MLF1IP, MYBL2, NCAPG, NCAPG2, NCAPH, NDC80, NEK2, NUP93, NUSAP1, OIP5, PBK, PDSS1, PKMYT1, PLK1, PLK4, PRC1, PTTG1, RACGAP1, RAD51, RAD51AP1, RAI2, RFC4, RPLPO, SETBP1, SF3B3, SHMT2, SLC25A12, SPAG5, SPC25, SQLE, STARD13, STIL, STMN1, SYNC, TACC3, TK1, TOP2A, TPX2, TRIP13, TROAP, TTK, UBE2C, UBE2S, ZWINT, C10orf76, C12orf72, CIAO1, CNOT4, DBR1, DND1, FBXO42, GRK4, HNRNPK, HNRNPL, HNRNPR, KHDRBS1, KLRAQ1, LACE1, LOC148189, LOC285033, LOC493754, MRPL44, NRF1, PKNOX1, PPHLN1, RRN3P3, SENP8, SLC4A1AP, TARDBP, THRAP3, TTLL11, WDR33 und ZNF143 besteht.

## Revendications

1. Méthode permettant de déterminer un pronostic du cancer du sein, la méthode comprenant les étapes de :

(a) mesure des niveaux d'expression d'un premier groupe de gènes, d'un deuxième groupe de gènes, d'un troisième groupe de gènes, d'un quatrième groupe de gènes, d'un cinquième groupe de gènes et d'un sixième groupe de gènes dans un échantillon biologique obtenu auprès d'un sujet, et la normalisation des niveaux d'expression mesurés ;

(b) calcul d'un indice de décision (DI) en multipliant le niveau d'expression de chacun du premier groupe de gènes, du deuxième groupe de gènes, du troisième groupe de gènes, du quatrième groupe de gènes, du cinquième groupe de gènes et du sixième groupe de gènes, normalisé à l'étape (a), par une valeur (pondérée) de coefficient de régression, et en additionnant les valeurs multipliées, ce qui permet de prédire le pronostic du cancer du sein chez le sujet, et

(c) détermination que le pronostic du cancer du sein est mauvais lorsque la valeur de l'indice de décision (DI) est supérieure à 20,

dans laquelle :

le premier groupe de gènes est constitué par l'ESR1 (récepteur 1 des œstrogènes), le PGR (récepteur de la progestérone) et le SCUBE2 (protéine 2 contenant un peptide signal, un domaine CUB et un domaine de type EGF) ;

le deuxième groupe de gènes est constitué par la CTSL2 (cathepsine V) et la MMP11 (métallopeptidase matricielle 11) ;

le troisième groupe de gènes est constitué par le TFRC (récepteur de la transferrine) et le CX3CR1 (récepteur 1 de la chimiokine à motif C-X3-C) ;

le quatrième groupe de gènes est constitué par le KIF14 (membre de la famille de la kinésine 14), la RRM2 (sous-unité M2 de régulation de la ribonucléotide réductase), la SHCBP1 (protéine 1 associée à un fuseau et de liaison à SHC), le SLC7A5 (membre 5 de la famille 7 des transporteurs-solutés) et la KPNA2 (sous-unité de la caryophérine alpha 2) ;

le cinquième groupe de gènes est constitué par l'AURKA (kinase aurora A), la CCNE2 (cycline E2), la CENPE (protéine centromérique E), E2F8 (facteur 8 de transcription de l'E2F), le KIF18A (membre de la famille de la kinésine 18A) et le KIF23 (membre de la famille de la kinésine 23) ; et

le sixième groupe de gènes est constitué par la JMJD5 (lysine déméthylase 8), la CACNA1D (sous-unité alpha1 D du canal dépendant de la tension calcique) et la GSTM1 (glutathion S-transférase Mu 1),

ledit indice de décision (DI) étant exprimé par l'équation ci-dessous :

$$DI = 0{,}62 \times KIF23 + 0{,}60 \times SLC7A5 + 0{,}59 \times KPNA2 + 0{,}53 \times AURKA + 0{,}34 \times E2F8 + 0{,}34 \times MMP11 + 0{,}24 \times SHCBP1 + 0{,}20 \times CTSL2 + 0{,}16 \times CENPE + 0{,}16 \times TFRC + 0{,}15 \times KIF18A + 0{,}14 \times CCNE2 + 0{,}04 \times KIF14 + 0{,}04 \times RRM2 + (-0{,}04) \times CX3CR1 + (-0{,}05) \times JMJD5 + (-0{,}33) \times CACNA1D + (-0{,}36) \times ESR1 + (-0{,}48) \times GSTM1 + (-1{,}45) \times PGR + (-2{,}04) \times SCUBE2 + 41{,}16.$$

2. Procédé selon la revendication 1, ladite étape de normalisation des niveaux d'expression mesurés comprenant la normalisation du niveau d'expression de chaque groupe de gènes à l'aide d'un ou de plusieurs gènes de référence choisis dans le groupe constitué par ACTB, APOBEC3B, ASF1B, ASPM, AURKB, BAG1, BCL2, BIRC5, BLM, BUB1, BUB1B, C14orf45, C16orf61, C7orf63, CCNA2, CCNB1, CCNB2, CCNE1, CCT5, CD68, CDC20, CDC25A, CDC45, CDC6, CDCA3, CDCA8, CDK1, CDKN3, CENPA, CENPF, CENPM, CENPN, CEP55, CHEK1, CIRBP, CKS2, CRIM1, CYBRD1, DBF4, DDX39, DLGAP5, DNMT3B, DONSON, DTL, E2F1, ECHDC2, ERBB2, ERCC6L, ESPL1, EXO1, EZH2, FAM64A, FANCI, FBXO5, FEN1, FOXM1, GAPDH, GINS1, GRB7, GTSE1, GUSB, HJURP, HMMR, HN1, IFT46, KIF11, KIF15, KIF18B, KIF20A, KIF2C, KIF4A, KIFC1, LMNB1, LMNB2, LRIG1, LRRC48, LRRC59, MAD2L1, MARCH8, MCM10, MCM2, MCM6, MELK, MKI67, MLF1IP, MYBL2, NCAPG, NCAPG2, NCAPH, NDC80, NEK2, NUP93, NUSAP1, OIP5, PBK, PDSS1, PKMYT1, PLK1, PLK4, PRC1, PTTG1, RACGAP1, RAD51, RAD51AP1, RAI2, RFC4, RPLPO, SETBP1, SF3B3, SHMT2, SLC25A12, SPAG5, SPC25, SQLE, STARD13, STIL, STMN1, SYNC, TACC3, TK1, TOP2A, TPX2, TRIP13, TROAP, TTK, UBE2C, UBE2S, ZWINT, C10orf76, C12orf72, CIAO1, CNOT4, DBR1, DND1, FBXO42, GRK4, HNRNPK, HNRNPL, HNRNPR, KHDRBS1, KLRAQ1, LACE1, LOC148189, LOC285033, LOC493754, MRPL44, NRF1, PKNOX1, PPHLN1, RRN3P3, SENP8, SLC4A1AP, TARDBP, THRAP3, TTLL11, WDR33 et ZNF143.

3. Dispositif destiné à être utilisé pour déterminer un pronostic du cancer du sein, le dispositif comprenant :

(a) une unité de détection conçue pour mesurer les niveaux d'expression d'un premier groupe de gènes, d'un deuxième groupe de gènes, d'un troisième groupe de gènes, d'un quatrième groupe de gènes, d'un cinquième groupe de gènes et d'un sixième groupe de gènes dans un échantillon biologique obtenu auprès d'un sujet et normaliser les niveaux d'expression mesurés ;

(b) une unité arithmétique conçue pour calculer un indice de décision (DI) en multipliant le niveau d'expression de chacun du premier groupe de gènes, du deuxième groupe de gènes, du troisième groupe de gènes, du quatrième groupe de gènes, du cinquième groupe de gènes et du sixième groupe de gènes, normalisé dans l'unité de détection, par une valeur (pondérée) de coefficient de régression, et en additionnant les valeurs multipliées; et

(c) une unité de sortie conçue pour prédire le pronostic du cancer du sein chez le sujet par l'indice de décision obtenu dans l'unité arithmétique et sortir le résultat prédit,

dans lequel :

le premier groupe de gènes est constitué par l'ESR1 (récepteur 1 des œstrogènes), le PGR (récepteur de la progestérone) et le SCUBE2 (protéine 2 contenant un peptide signal, un domaine CUB et un domaine de type EGF) ;

le deuxième groupe de gènes est constitué par la CTSL2 (cathepsine V) et la MMP11 (métallopeptidase matricielle 11) ;

le troisième groupe de gènes est constitué par le TFRC (récepteur de la transferrine) et le CX3CR1 (récepteur 1 de la chimiokine à motif C-X3-C) ;

le quatrième groupe de gènes est constitué par le KIF14 (membre de la famille de la kinésine 14), la RRM2 (sous-unité M2 de régulation de la ribonucléotide réductase), la SHCBP1 (protéine 1 associée à un fuseau et de liaison à SHC), le SLC7A5 (membre 5 de la famille 7 des transporteurs-solutés) et la KPNA2 (sous-unité de la caryophérine alpha 2) ;

le cinquième groupe de gènes est constitué par l'AURKA (kinase aurora A), la CCNE2 (cycline E2), la CENPE (protéine centromérique E), E2F8 (facteur 8 de transcription de l'E2F), le KIF18A (membre de la famille de la kinésine 18A) et le KIF23 (membre de la famille de la kinésine 23) ; et

le sixième groupe de gènes est constitué par la JMJD5 (lysine déméthylase 8), la CACNA1D (sous-unité alpha1 D du canal dépendant de la tension calcique) et la GSTM1 (glutathion S-transférase Mu 1),

l'indice de décision (DI) étant exprimé par l'équation ci-dessous :

$$DI = 0,62 \times KIF23 + 0,60 \times SLC7A5 + 0,59 \times KPNA2 + 0,53 \times AURKA + 0,34 \times E2F8 + 0,34 \times MMP11 + 0,24 \times SHCBP1 + 0,20 \times CTSL2 + 0,16 \times CENPE + 0,16 \times TFRC + 0,15 \times KIF18A + 0,14 \times CCNE2 + 0,04 \times KIF14 + 0,04 \times RRM2 + (-0,04) \times CX3CR1 + (-0,05) \times JMJD5 + (-0,33) \times CACNAID + (-0,36) \times ESR1 + (-0,48) \times GSTM1 + (-1,45) \times PGR + (-2,04) \times SCUBE2 + 41,16$$

ladite unité de sortie déterminant que le pronostic du cancer du sein est mauvais lorsque la valeur de l'indice de décision (DI) est supérieure à 20.

4. Dispositif selon la revendication 3, ladite unité de détection normalisant les niveaux d'expression du premier groupe de gènes, du deuxième groupe de gènes, du troisième groupe de gènes, du quatrième groupe de gènes, du cinquième groupe de gènes et du sixième groupe de gènes à un niveau d'expression d'un gène de référence, ledit gène de référence étant un ou plusieurs gènes sélectionnés dans le groupe constitué par ACTB, APOBEC3B, ASF1B, ASPM, AURKB, BAG1, BCL2, BIRC5, BLM, BUB1, BUB1B, C14orf45, C16orf61, C7orf63, CCNA2, CCNB1, CCNB2, CCNE1, CCT5, CD68, CDC20, CDC25A, CDC45, CDC6, CDCA3, CDCA8, CDK1, CDKN3, CENPA, CENPF, CENPM, CENPN, CEP55, CHEK1, CIRBP, CKS2, CRIM1, CYBRD1, DBF4, DDX39, DLGAP5, DNMT3B, DONSON, DTL, E2F1, ECHDC2, ERBB2, ERCC6L, ESPL1, EXO1, EZH2, FAM64A, FANCI, FBXO5, FEN1, FOXM1, GAPDH, GINS1, GRB7, GTSE1, GUSB, HJURP, HMMR, HN1, IFT46, KIF11, KIF15, KIF18B, KIF20A, KIF2C, KIF4A, KIFC1, LMNB1, LMNB2, LRIG1, LRRC48, LRRC59, MAD2L1, MARCH8, MCM10, MCM2, MCM6, MELK, MKI67, MLF1IP, MYBL2, NCAPG, NCAPG2, NCAPH, NDC80, NEK2, NUP93, NUSAP1, OIP5, PBK, PDSS1, PKMYT1, PLK1, PLK4, PRC1, PTTG1, RACGAP1, RAD51, RAD51AP1, RAI2, RFC4, RPLP0, SETBP1, SF3B3, SHMT2, SLC25A12, SPAG5, SPC25, SQLE, STARD13, STIL, STMN1, SYNC, TACC3, TK1, TOP2A, TPX2, TRIP13, TROAP, TTK, UBE2C, UBE2S, ZWINT, C10orf76, C12orf72, CIAO1, CNOT4, DBR1, DND1, FBXO42, GRK4, HNRNPK, HNRNPL, HNRNPR, KHDRBS1, KLRAQ1, LACE1, LOC148189, LOC285033, LOC493754, MRPL44, NRF1, PKNOX1, PPHLN1, RRN3P3, SENP8, SLC4A1AP, TARDBP, THRAP3, TTLL11, WDR33, et ZNF143.

[FIG. 1a]

[FIG. 1b]

[FIG. 2]

## Distant Metastasis-Free Survival (N=413)

Decision Index™≤ 20

Decision Index™ > 20

## Metastasis rate according to DI

| | Low (DI ≤20) (N=269, 65.1%) | High (DI >20) (N=144, 34.9%) |
|---|---|---|
| Distant Metastasis (N) | 20 (7.4%) | 57 (39.6%) |
| **5-year Metastasis-free Survival** | **97.03 % (94.14, 98.50)** | **77.08 % (69.32, 83.12)** |
| 10-year Metastasis-free Survival | 92.63 % (88.25, 95.42) | 61.62 % (52.22, 69.71) |
| Log-rank (p-value) | <0.0001 | |
| **Hazard Ratio (p-value)** | **6.568 (3.938, 10.956); <0.0001** | |

[FIG. 3a]

## Distant Metastasis-Free Survival (Age ≤ 50, N=260)

Decision Index™ ≤ 20

Decision Index™ > 20

63.0% (260/413) of patients at Age≤50

| Age ≤ 50 | Low (DI ≤20) (N=176, 67.7%) | High (DI >20) (N=84, 32.3%) |
|---|---|---|
| Distant Metastasis (N) | 11 (6.3%) | 32 (38.1%) |
| 5-year Metastasis-free Survival | 97.73 % (94.06, 99.14) | 78.57% (68.17, 85.92) |
| 10-year Metastasis-free Survival | 93.60 % (87.96, 96.83) | 58.96 % (45.46, 70.18) |
| Log-rank (p-value) | <0.0001 | |
| Hazard Ratio (p-value) | 7.794 (3.919, 15.502) (<0.0001) | |

[FIG. 3b]

**Distant Metastasis-Free Survival (Age > 50, N=153)**

| Age > 50 | Low (DI ≤20) (N=93, 60.8%) | High (DI >20) (N=60, 39.2%) |
|---|---|---|
| Distant Metastasis (N) | 9 (9.7%) | 25 (41.7%) |
| 5-year Metastasis-free Survival | 95.70 % (88.95, 98.36) | 75.00 % (62.00, 84.10) |
| 10-year Metastasis-free Survival | 89.98 % (80.77, 94.92) | 64.06 % (50.28, 74.95) |
| Log-rank (p-value) | <0.0001 | |
| Hazard Ratio (p-value) | 5.114 (2.379, 10.994) (<0.0001) | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150079591 A1

- KR 20180052985 A

**Non-patent literature cited in the description**

- **FISHER et al.** *Lancet*, vol. 10 (364), 858-68
- **PAIK et al.** *J Clin Oncol*, vol. 24 (23), 3726-34
- **WKILLIAMS AD et al.** *Ann Surg Oncol.*, October 2018, vol. 25 (10), 2875-2883
- **GUNHEE LEE** ; **MINHO LEE**. *Genomics & Informatics*, 2017, vol. 15, 156-161
- **COLLIGNON OLIVIER et al.** *Plos One*, 2018, 13
- **LIU MIN et al.** *Breast Cancer*, 2019, vol. 26, 317-325
- *International Consensus Panel of the St. Gallen Conference*, 2001
- **GOLDHIRSCH et al.** *J. Clin. Oncol.*, vol. 19, 3817-3827
- **ROBINSON et al.** *Bioinformatics*, 2010
- **DILLIES et al.** *Briefings in bioinformatics*, 2013
- **DOBIN et al.** *Bioinformatics*, 2013
- **ANDERS et al.** *Bioinformatics*, 2014